# EUROPÄISCHE PATENTSCHRIFT

(11) **EP 1 142 902 B1**
(45) Veröffentlichungstag und Bekanntmachung des Hinweises auf die Patenterteilung: **26.10.2005**
(21) Anmeldenummer: 01116471.2
(22) Anmeldetag: 21.01.1993
(51) Int. Cl.: C07H 21/00, C07F 9/24, A61K 31/7125, C12Q 1/68, A61P 31/12, A61P 35/00

(54) **3'-Derivatisierte Oligonucleotidanaloga mit nichtnucleotidischen Gruppierungen, deren Herstellung und Verwendung**
3'-derivatised oligonucleotide analogues with non-nucleotidic groups, their preparation and use
Analogues des oligonucléotides dérivées en position 3', avec des groupes non-nucléotidiques, leur préparation et leur utilisation

(30) Priorität: 22.01.1992 DE 4201663
(43) Veröffentlichungstag der Anmeldung: 10.10.2001
(62) Teilanmeldung aus: 93100893.2
(73) Patentinhaber: Aventis Pharma Deutschland GmbH, 65929 Frankfurt am Main (DE)
(72) Erfinder: Peyman, Anuschirwan, Dr., 65779 Kelkheim (DE); Uhlmann, Eugen, Dr., 61479 Glashütten (DE); Winkler, Irvin, Dr., 65835 Liederbach (DE)

(56) Entgegenhaltungen:
- EP-A- 0 289 619
- WO-A-89/12060
- WO-A-91/06556
- WO-A-92/02638
- WO-A-92/06103
- N.G.DOLINNAYA ET AL.: "Site-Directed Modification of DNA Duplexes by Chemical Ligation." NUCLEIC ACIDS RESEARCH., Bd. 16, Nr. 9, 1988, Seiten 3721-3738, XP002175408 ARLINGTON, VIRGINIA US
- J.GOODCHILD: "Conjugates of Oligonucleotides and Modified Oligonucleotides: A Review of Their Synthesis and Properties." BIOCONJUGATE CHEMISTRY, Bd. 1, Nr. 3, 1990, Seiten 165-187, XP000174627
- E.UHLMANN ET AL.: "Antisense Oligonucleotides: A New Therapeutic Principle." CHEMICAL REVIEWS, Bd. 90, Nr. 4, 1990, Seiten 543-584, XP000141412 WASHINGTON
- N.T.THUONG ET AL.: "Nouvelle Méthode de préparation d'esters Phosphoriques, Renfermant un Groupe B-Mercaptoéthyle, Utilisables en Synthèse Nucléotidique." BULLETIN DE LA SOCIETE CHIMIQUE DE FRANCE. 2 PARTIE - CHIMIE ORGANIQUE, BIOCHIMIE, Nr. 1-2, 1981, Seiten II-51-II-56, XP002175409 PARIS FR

## Beschreibung

Die vorliegende Erfindung betrifft neue Oligonucleotidanaloga mit wertvollen physikalischen, biologischen und pharmakologischen Eigenschaften sowie ein Verfahren zu deren Herstellung. Ihre Anwendung bezieht sich auf die Verwendung als Inhibitoren der Genexpression (Antisense Oligonucleotide, Ribozyme, Sense Oligonucleotide und Triplex Forming Oligonucleotide), als Sonden zum Nachweis von Nucleinsäuren und als Hilfsmittel in der Molekularbiologie.
Oligonucleotide finden in wachsendem Maße Anwendung als Inhibitoren der Genexpression (G. Zon, Pharmaceutical Research 5, 539 (1988); J. S. Cohen, Topics in Molecular and Structural Biology 12 (1989) Macmillan Press; C. Helene and J. J. Toulme, Biochemica et Biophysica Acta 1049, 99 (1990); E. Uhlmann and A. Peyman, Chemical Reviews 90, 543 (1990)). Antisense Oligonucleotide sind Nucleinsäure-Fragmente, deren Basensequenz komplementär ist zu einer zu inhibierenden mRNA. Diese Target-mRNA kann zellulären, viralen oder sonstigen pathogenen Ursprungs sein. Als zelluläre Target-Sequenzen kommen beispielsweise die von Rezeptoren, Enzymen, Immunmodulatoren, lonenkanälen oder Onkogenen in Frage. Die Inhibition der Virus Vermehrung mit Hilfe von Antisense Oligonucleotiden wurde beispielsweise für RSV (Rous Sarcoma Virus), HSV-1 und -2 (Herpes Simplex Virus Typ I und II), HIV (Human Immunodeficiency Virus) und Influenza-Viren beschrieben. Dabei setzt man Oligonucleotide ein, die zur viralen Nucleinsäure komplementär sind. Sense Oligonucleotide sind dagegen in ihrer Sequenz so konzipiert, daß sie beispielsweise Nucleinsäure-bindende Proteine oder Nucleinsäure-prozessierende Enzyme binden ("einfangen") und so deren biologische Aktivität inhibieren (Helene, 1990). Als virale Targets sind hier beispielsweise die Reverse Transkriptase, DNA-Polymerase und Transaktivator-Proteine zu nennen. Triplex Forming Oligonucleotide haben im allgemeinen die DNA als Target und bilden nach Bindung an diese eine tripelhelicale Struktur aus. Während mit Hilfe der Antisense Oligonucleotide im allgemeinen die Prozessierung (Splicing etc.) der mRNA oder deren Translation in das Protein gehemmt werden, hemmen Triplex Forming Oligonucleotide die Transkription oder Replikation der DNA (Helene et al., 1990, Uhlmann und Peyman, 1990). Es ist aber auch möglich, einzelsträngige Nucleinsäuren in einer ersten Hybridisierung mit einem Antisense Oligonucleotid unter Ausbildung eines Doppelstranges zu binden, der dann in einer zweiten Hybridisierung mit einem Triplex Forming Oligonucleotid eine Triplex-Struktur ausbildet. Die Antisense und Triplex Bindungsregionen können dabei entweder in zwei separaten Oligonucleotiden oder aber in einem Oligonucleotid beherbergt sein. Eine weitere Anwendung synthetischer Oligonucleotide sind die sogenannten Ribozyme, welche die Target-RNA infolge ihrer Ribonuclease-Aktivität zerstören (J.J. Rossi and N. Sarver, TIBTECH 8, 179 (1990).
In der DNA-Diagnostik werden Nucleinsäure-Fragmente mit geeigneter Markierung als sogenannte DNA-Sonden oder DNA-Probes für die spezifische Hybridisierung an eine nachzuweisende Nucleinsäure eingesetzt. Die spezifische Ausbildung des neuen Doppelstranges wird dabei mit Hilfe der Markierung, die vorzugsweise nicht radioaktiv ist, verfolgt. Auf diese Weise lassen sich genetische, maligne, virale oder durch andere Pathogene verursachte Krankheiten nachweisen.

Für die meisten genannten Anwendungen sind Oligonucleotide in ihrer natürlich vorkommenden Form wenig oder völlig ungeeignet. Sie müssen chemisch so modifiziert werden, daß sie den speziellen Anforderungen gerecht werden. Damit Oligonucleotide in biologischen Systemen, beispielsweise zur Inhibition der Virus-Vermehrung eingesetzt werden können, müssen sie folgende Voraussetzungen erfüllen:
1. Sie müssen unter in vivo Bedingungen, also sowohl im Serum als auch intrazellulär, eine ausreichend große Stabilität aufweisen.
2. Sie müssen so beschaffen sein, das sie die Zell- und Nucleus-Membran passieren können.
3. Sie müssen unter physiologischen Bedingungen in Basen-spezifischer Weise an ihre Target-Nucleinsäure binden, um den inhibitorischen Effekt zu entfalten.

Für DNA-Sonden sind diese Voraussetzungen nicht unabdingbar; jedoch müssen diese Oligonucleotide so derivatisiert sein, daß ein Nachweis, beispielsweise mittels Fluoreszenz, Chemilumineszenz, Kolorimetrie oder spezifischer Färbung, möglich ist (Beck und Köster, Anal. Chem. 62, 2258 (1990).

Die chemische Veränderung der Oligonucleotide erfolgt meistens in der Weise, daß Phosphatrückgrat, Ribose-Einheit oder die Nucleobasen entsprechend verändert werden (Cohen, 1989; Uhlmann und Peyman, 1990). Eine weitere häufig genutzte Methode ist die Herstellung von Oligonucleotid-5'-Konjugaten durch Umsetzung der 5'-Hydroxy-Gruppe mit entsprechenden Phosphorylierungs-Reagenzien.
Oligonucleotide, die nur am 5'-Ende modifiziert sind, haben den Nachteil, daß sie im Serum abgebaut werden. Wenn dagegen alle Internucleotid-Phosphat-Reste verändert werden, verändern sich die Eigenschaften der Oligonuleotide oft drastisch.
Beispielsweise ist die Löslichkeit der Methylphosphonat Oligonucleotide in wäßrigem Medium vermindert und das Hybridisierungsvermögen reduziert.
Phosphorothioat-Oligonucleotide wirken unspezifisch, so daß beispielsweise auch Homooligomere gegen Viren wirksam sind.

Antisense Oligonucleotide besitzen im allgemeinen eine einheitliche Polarität, die bei der Hybridisierung an RNA meist antiparallelen Charakter aufweist (s. Tabelle 1; A). In bestimmten Fällen, beispielsweise bei aus a-Nucleosid-Einheiten aufgebauten Oligonucleotiden, kann die Polarität auch parallel sein (Tabelle 1; B). Triplex Forming Oligonucleotide können an doppelsträngige Nucleinsäuren im allgemeinen sequenzabhängig in paralleler (Tabelle 1; C) oder antiparalleler (Tabelle 1; D) Orientierung bezüglich des Purin-reichen Nucleinsäure-Stranges hybridisieren. Dazu werden hauptsächlich die Basenpaarungsmotive T●AT, G●GC, C⁺●GC, G●TA, C^{Me}●GC, A●AT und C^{Pi}●GC benutzt, worin C⁺ einen protonierten Cytosin-Rest, C^{Me} einen 5-Methyl-cytosin-Rest, C^{PI} einen Pseudoiso-cytosin-Rest und "●" eine Hoogsteen oder reverse Hoogsteen-Basenpaarung bedeuten. Jedoch ist die Anwendung dieser Hoogsteen-Basenpaarungen auf purinreiche Regionen der doppelsträngigen Nucleinsäuren beschränkt.

Um die Flexibilität der Triplex Forming Oligonucleotide bezüglich purinreicher Zielsequenzen zu erhöhen, wurden Oligonucleotide mit wechselnder Polarität hergestellt, die aufgrund eines (5'5')-Strangwechsels (Tabelle 1; E) oder eines (3'3')-Strangwechsels, die sich gegebenenfalls abwechseln können (Tabelle 1; F), jeweils purinreiche Bereiche des gegenüberliegenden Stranges binden können (Ono et al., Biochemistry (1991) 30, 9914).
Ein Strangwechsel unter Beibehaltung der Polarität ist möglich, wenn man einen (3'5')-Spacer einbaut.

Weiterhin ist es möglich, mit Hilfe von (5'5')-Schleifen (Tabelle 1; G) oder (3'5') oder (2'5')-Schleifen spezielle Antisense Oligonucleotide herzustellen, in denen eine Region durch Watson-Crick Basenpaarung einen Nucleinsäure-Einzelstrang und eine zweite Region einen Nucleinsäure-Doppelstrang, der sich aus der Watson-Crick Basenpaarung ergibt, durch Hoogsteen Basenpaarung erkennen. Mit Hilfe solcher Antisense/Triplex Oligonucleotidanaloga ist daher eine Triplexbildung an einzelsträngigen Nucleinsäuren möglich. In ähnlicher Weise lassen sich doppelsträngige Sense Oligonucleotide, die DNA-bindende Proteine sequenzspezifisch binden, über intramolekulare Watson-Crick Basenpaarungen herstellen (Tabelle 1; I).

Schließlich lassen sich Oligonucleotide herstellen, die am 5'-Ende einen 5'5'-Spacer (Tabelle 1; K) enthalten. In diesen Oligonucleotidanaloga können vorteilhaft beide 3'(2')-Enden Phosphorylgruppen enthalten.

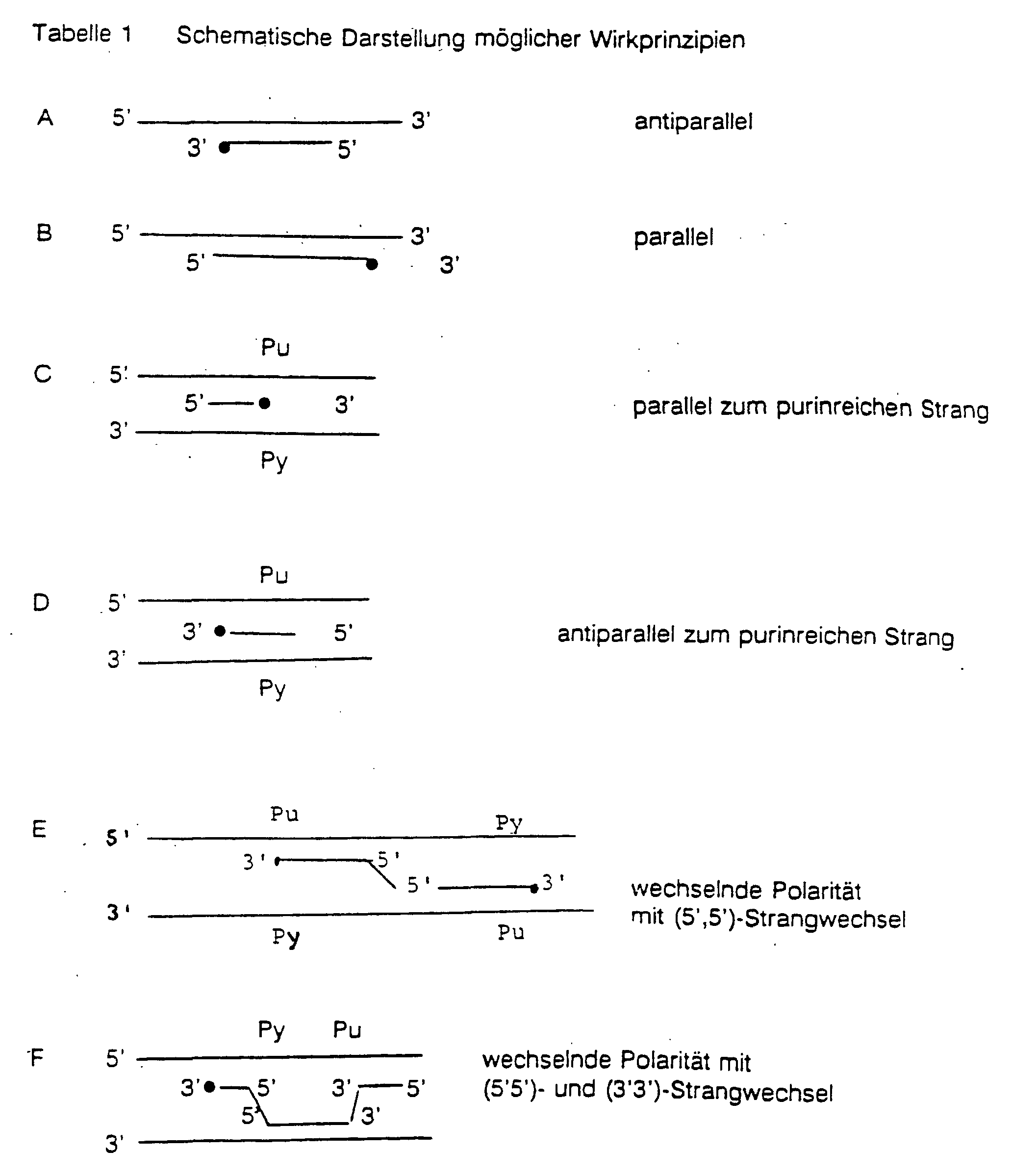

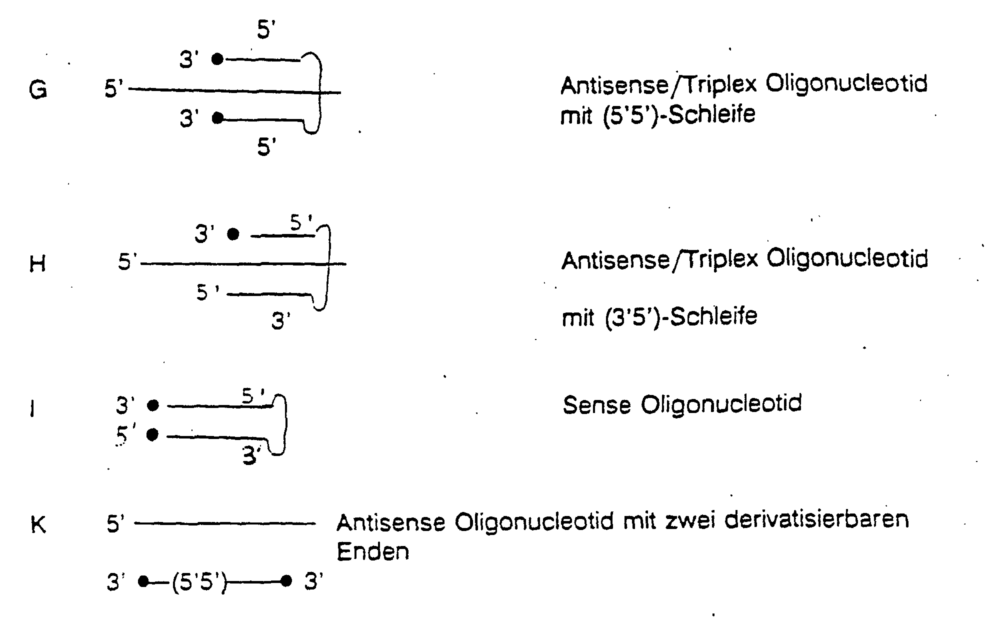

Die bislang bekannten Oligonucleotidanaloga, die den in Tabelle 1 C bis I dargestellten Wirkprinzipien gehorchen, besitzen im allgemeinen eine Hydroxygruppe am 3'-Ende, so daß sie im Serum abgebaut werden, meistens schlecht membrangängig sind und nur am 5'-Ende leicht derivatisierbar sind.

Aufgabe ist es daher, Oligonucleotidanaloga mit spezifischen Hybridisierungseigenschaften gegen einzel- und doppelsträngige Nucleinsäuren, erhöhter Serumstabilität, guter Löslichkeit und spezifischer Wirksamkeit bereitzustellen.

Gegenstand der Erfindung sind Oligonucleotidanaloga der Formel IB 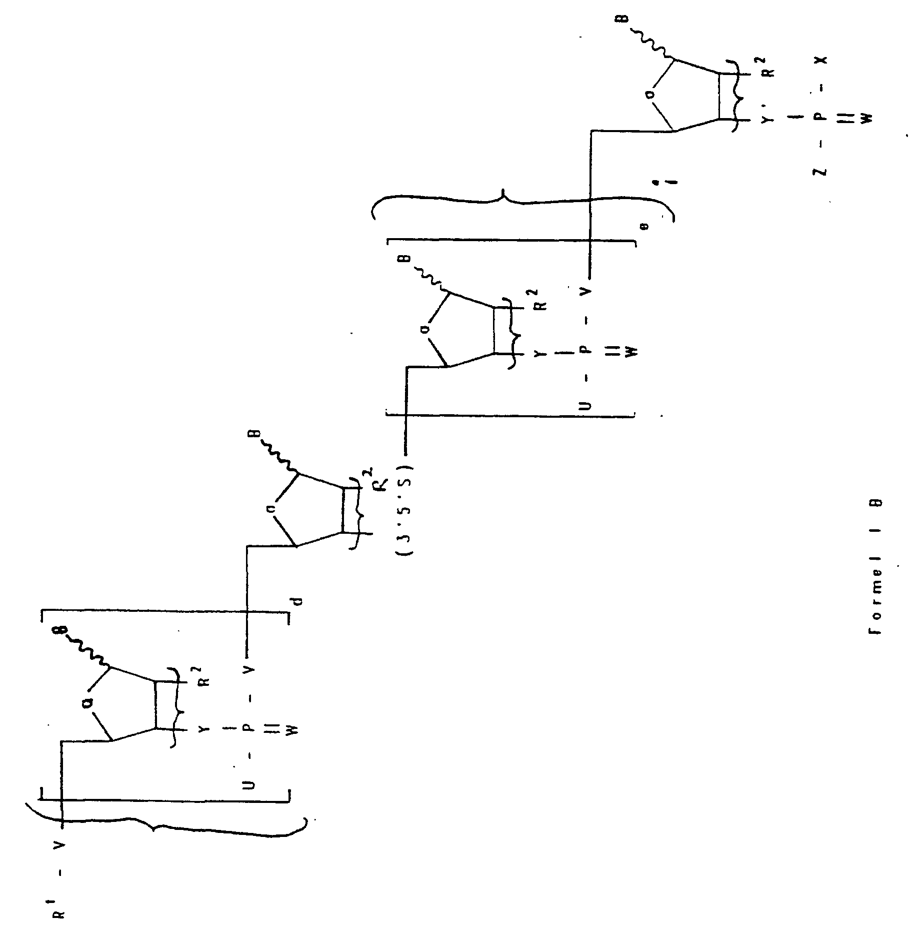 sowie deren physiologisch verträglichen Salze, worin
- R¹: Wasserstoff, C₁-C₁₈-Alkyl, vorzugsweise C₁-C₆-Alkyl, C₂-C₁₈-Alkenyl, C₂-C₁₈. Alkinyl, C₂-C₁₈-Alkylcarbonyl, C₃-C₁₉-Alkenylcarbonyl, C₃-C₁₉-Alkinylcarbonyl, C₆-C₂₀-Aryl, (C₆-C₁₄)-Aryl-(C₁-C₈)-alkyl, oder einen Rest der Formel II 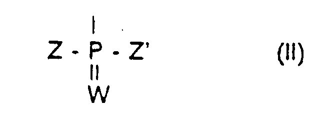
bedeutet;
- R²: Wasserstoff, Hydroxy, C₁-C₁₈-Alkoxy, Halogen, Azido oder NH₂ bedeutet;
- B: für eine in der Nucleotidchemie übliche Base, beispielsweise für natürliche Basen wie Adenin, Cytosin, Guanin und Thymin oder unnatürliche Basen wie Purin, 2.6-Diaminopurin, 7-Deazaadenin, 7-Deazaguanin, N⁴N⁴-Ethanocytosin, N⁶N⁶-Ethano-2.6-diaminopurin, 5-Methylcytosin, Pseudoisocytosin, steht;
- a: für Oxy oder Methylen steht;
- d,e,f: unabhängig voneinander eine ganze Zahl von 0 bis 50, bevorzugt 5 bis 15, bedeuten;
- i: eine ganze Zahl von 1 bis 10, vorzugsweise 1 bis 3, ist;
- W: Oxo, Selenoxo oder Thioxo bedeutet;
- V: Oxy, Sulfandiyl oder Imino bedeutet;
- Y: Oxy, Sulfandiyl, Imino oder Methylen bedeutet;
- Y': Oxy, Sulfandiyl, Imino, (CH₂)ₘ oder V(CH₂)ₘ bedeutet, worin
- m: eine ganze Zahl von 1 bis 18, vorzugsweise von 1 bis 6, bedeutet;
- X: Hydroxy oder Mercapto bedeutet;
- U: Hydroxy, Mercapto, SeH, C₁-C₁₈-Alkoxy, vorzugsweise C₁-C₆-Alkoxy, C₁-C₁₈-Alkyl,vorzugsweise C₁-C₆-Alkyl, C₆-C₂₀-Aryl, (C₆-C₁₄)-Aryl-(C₁-C₈)-alkyl, NHR³, NR³R⁴ oder einen Rest der Formel (OCH₂CH₂)ₚO(CH₂)_{q}CH₂R¹¹ bedeutet, worin
- R³: C₁-C₁₈-Alkyl, vorzugsweise C₁-C₈-Alkyl, C₆-C₂₀-Aryl, (C₆-C₁₄)-Aryl-(C₁-C₈)-alkyl, -(CH₂)_{c}-[NH(CH₂)_{c}]_{d}-NR¹²R¹², worin c eine ganze Zahl von 2 bis 6 und d eine ganze Zahl von 0 bis 6 ist, und R¹² unabhängig voneinander Wasserstoff oder C₁-C₆Alkyl oder C₁-C₄-Alkoxy-C₁-C₆-alkyl, bevorzugt Methoxyethyl, ist;
- R⁴: C₁-C₁₈-Alkyl, vorzugsweise C₁-C₈-Alkyl und besonders bevorzugt C₁-C₄-Alkyl, C₆-C₂₀-Aryl oder (C₆-C₁₀)-Aryl-(C₁-C₈)-alkyl bedeutet oder im Falle von NR³R⁴ zusammen mit R³ und dem sie tragenden Stickstoffatom einen 5-6-gliedrigen heterozyklischen Ring bedeutet, der zusätzlich ein weiteres Heteroatom aus der Reihe O, S, N enthalten kann,
- p: eine ganze Zahl von 1 bis 100, bevorzugt 3 bis 20 und besonders bevorzugt 3 bis 8, ist,
- q: eine ganze Zahl von 0 bis 18, bevorzugt 0 bis 15, ist,
- R¹¹: Wasserstoff oder eine funktionelle Gruppe wie Hydroxy, Amino, NHR¹³, COOH, CONH₂, COOR¹² oder Halogen bedeutet, worin R¹² C₁-C₄-Alkyl, vorzugsweise Methyl, bedeutet;
- S: eine Gruppe der Formel III ist 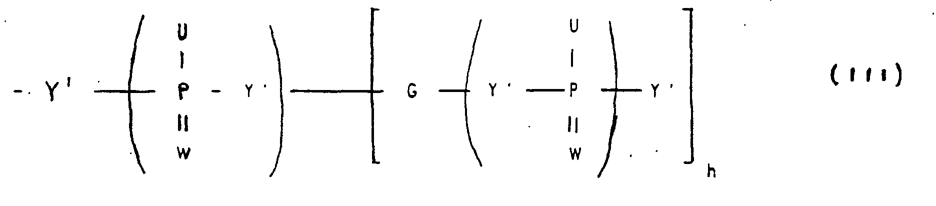
worin
- h: vier oder fünf ist;
- G: die Bedeutung hat von C₁-C₁₂-Alkylen, insbesondere C₂-C₄-Alkylen, wobei Alkylen gegebenenfalls durch Halogen, Amino, Hydroxy, C₁-C₁₈-Alkyl, C₁-C₁₈-Alkoxy, C₁-C₁₈-Alkylcarbonyloxy, C₆-C₁₄-Aryl, C₆-C₁₄-Aryl-C₁-C₁₈-alkyl, oder C₆-C₁₄-Aryl-C₁-C₈-alkoxy substituiert sein kann, C₆-C₁₄-Aryl-di-C₁-C₈-alkylen, C₆-C₁₈-Arylen einer Gruppe der Formel (CH₂CH₂V)_{α}CH₂CH₂ oder (CH₂V)_{α}CH₂, worin α eine ganze Zahl von 1 bis 11, vorzugsweise von 1 bis 5 ist,
einer Einheit der Formel 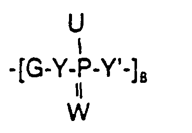 worin β eine ganze Zahl von 1 bis 6 bedeutet, oder einer Gruppe der Formel 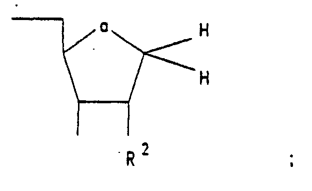
- Z= Z': Hydroxy, Mercapto, SeH, C₁-C₂₂-Alkoxy, vorzugsweise C₆-C₁₈-Alkoxy, -O-(CH₂)_{b}-NR¹²R¹³, worin b eine ganze Zahl von 1 bis 6 ist, und R¹³ C₁-C₆-Alkyl ist oder R¹² und R¹³ zusammen mit dem sie tragenden Stickstoffatom einen 3-6 gliedrigen Ring bilden, C₁-C₁₈-Alkyl, vorzugsweise C₁-C₈-Alkyl, C₆-C₂₀-Aryl, (C₆-C₁₄)-Aryl-(C₁-C₈)-alkyl, vorzugsweise (C₆-C₁₀)-Aryl-(C₁-C₄)-alkyl, (C₆-C₁₄)-Aryl-(C₁-C₈)-alkoxy, vorzugsweise (C₆-C₁₀)-Aryl-(C₁-C₄)-alkoxy, wobei Aryl auch Heteroaryl bedeutet und Aryl gegebenenfalls durch 1, 2 oder 3 gleiche oder verschiedene Reste aus der Reihe Carboxy, Amino, Nitro, C₁-C₄-Alkylamino, Hydroxy, Halogen und Cyano substituiert ist, C₁-C₁₈. Alkylmercapto, NHR³, NR³R⁴, einen Rest der Formel III oder eine Gruppe bedeuten, die die intrazelluläre Aufnahme begünstigt oder als Markierung einer DNA-Sonde dient;
die geschweifte Klammer in 2'- und 3'-Stellung andeutet, daß sich R² und der benachbarte Phosphorylrest auch umgekehrt in 3'- und 2'-Stellung befinden können,
wobei jedes Nucleotid in seiner D- bzw. L-Konfiguration vorliegen kann und sich die Base B in α- bzw. β-Stellung befinden kann.

Bevorzugt sind Oligonucleotidanaloga der Formel IB, worin sich die Base B in β-Stellung befindet, die Nucleotide in der D-Konfiguration vorliegen, R² sich in 2'-Stellung befindet und a für Oxy steht.

Besonders bevorzugt sind Oligonucleotidanaloga der Formel IB, worin
- R¹: Wasserstoff, C₁-C₆-Alkyl, insbesondere Methyl, oder einen Rest der Formel II bedeutet;
- R²: Wasserstoff oder Hydroxy, insbesondere Wasserstoff, bedeutet;
- d,e und f: eine ganze Zahl von 5 bis 15 bedeuten;
- i: eine ganze Zahl von 1 bis 3 bedeutet;
- m: eine ganze Zahl von 1 bis 6, insbesondere 1, bedeutet;
- U: Hydroxy, Mercapto, C₁-C₆-Alkoxy, C₁-C₆-Alkyl, NR³R⁴ oder NHR³, insbesondere Hydroxy oder C₁-C₆-Alkyl, bedeutet, worin
- R³: C₁-C₈-Alkyl, bevorzugt C₁-C₄-Alkyl, oder Methoxyethyl ist, und B, W, V, Y, Y', X und Z die obengenannte Bedeutung haben.

Insbesondere bevorzugt sind Oligonucleotidanaloga der Formel IB, worin V, Y' und Y die Bedeutung von Oxy haben.
Weiterhin besonders bevorzugt sind Oligonucleotidanaloga der Formel IB, worin V, Y, Y' und W die Bedeutung von Oxy bzw. Oxo haben.
Ganz besonders bevorzugt sind Oligonucleotidanaloga der Formel IB, worin V, Y, Y', W und U die Bedeutung von Oxy, Oxo bzw. Hydroxy haben.
Ferner sind Oligonucleotidanaloga der Formel IB bevorzugt, worin R¹ für Wasserstoff steht.
Insbesondere bevorzugt sind Oligonucleotidanaloga der Formel IB, worin U, V, W, X, Y' und Y die Bedeutung von Oxy, Oxo bzw. Hydroxy haben und R¹ gleich Wasserstoff ist.

Die wiederholt auftretenden Reste wie R², B, a, d, e, f, h, W, V, Y, Y', U, R³, R⁴, p, q, G und Z können unabhängig voneinander gleiche oder verschiedene Bedeutungen haben, d.h. z.B. V bedeutet unabhängig voneinander Oxy, Sulfandiyl oder Imino.
Halogen steht vorzugsweise für Fluor, Chlor oder Brom.
Unter Heteroaryl ist der Rest eines monocyclischen oder bicyclischen (C₃-C₉)-Heteroaromaten zu verstehen, der im Ringsystem ein oder zwei N-Atome und/oder ein S- oder ein O-Atom enthält.
Beispielhaft für Gruppen, die die intrazelluläre Aufnahme begünstigen, sind verschiedene lipophile Reste wie -O-(CH₂)ₓ-CH₃, worin x eine ganze Zahl von 6-18 bedeutet, -C-(CH₂)ₙ-CH=CH-(CH₂)ₘ-CH₃, worin n und m unabhängig voneinander eine ganze Zahl von 6 bis 12 bedeuten, -O-(CH₂CH₂O)₄-(CH₂)₉-CH₃, -O-(CH₂CH₂O)₈. (CH₂)₁₃-CH₃ und -O-(CH₂CH₂O)₇-(CH₂)₁₅-CH₃, aber auch Steroid-Reste wie Cholesteryl und Konjugate, die natürliche Carriersysteme ausnutzen wie Gallensäure, Folsäure, 2-(N-Alkyl, N-Alkoxy)-Aminoanthrachinon und Konjugate der Mannose und Peptide der entsprechenden Rezeptoren, die zur rezeptorvermittelten Endozytose der Oligonucleotide führen wie EGF (Epidermal Growth Factor), Bradykinin und PDGF (Platelet Derived Growth Factor). Unter Markierungs-Gruppen sind fluoreszierende Gruppen beispielsweise von Dansyl-(=N-Dimethyl-1-aminonaphthyl-5-sulfonyl-), Fluorescein- oder Coumarin-Derivaten oder chemilumineszierende Gruppen beispielsweise von Acridin-Drivaten zu verstehen sowie das über ELISA nachweisbare Digoxygenin-System, die über das Biotin/Avidin-System nachweisbare Biotin-Gruppe oder aber Linker-Arme mit funktionellen Gruppen, die eine nachträgliche Derivatisierung mit nachweisbaren Reporter-Gruppen gestatten, beispielsweise ein Aminoalkyl-Linker, der mit einem Acridinium-Aktivester zur Chemilumineszenz-Probe umgesetzt wird. Typische Markierungsgruppen sind:

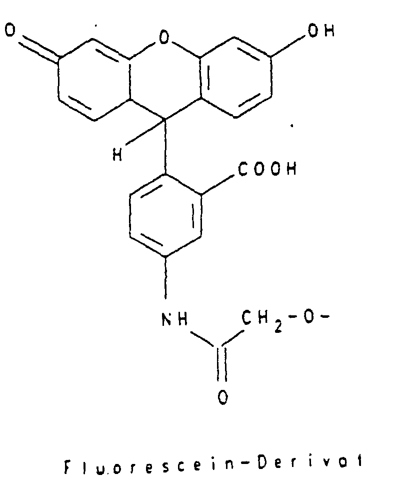

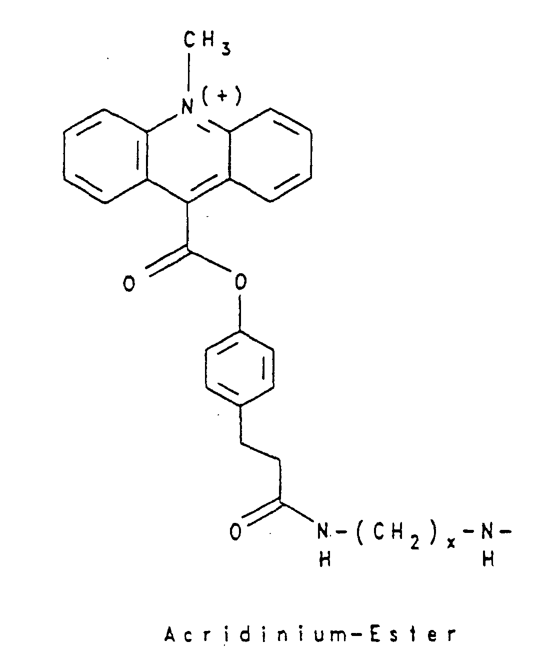

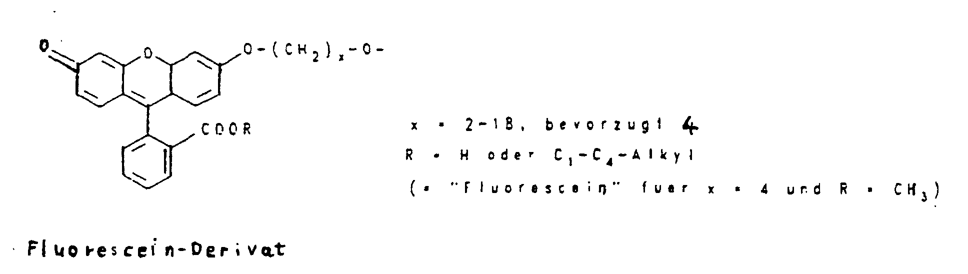

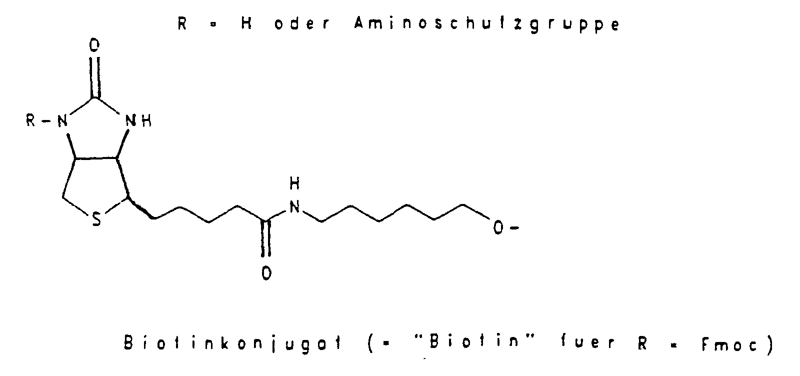

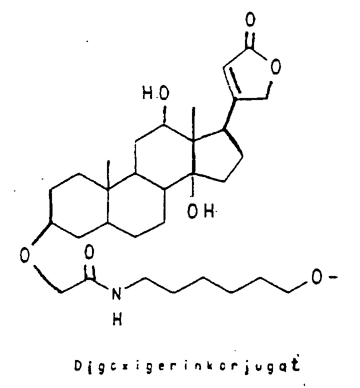

Oligonucleotidanaloga, die an Nucleinsäuren binden bzw. interkalieren und/oder spalten oder quervernetzen, enthalten z. B. Acridin-, Psoralen-, Phenanthridin, Naphtochinon-, Daunomycin- oder Chlorethylaminoaryl-Konjugate. Typische interkalierende und quervernetzende Reste sind:

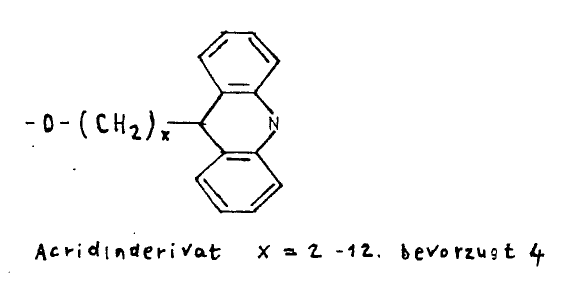

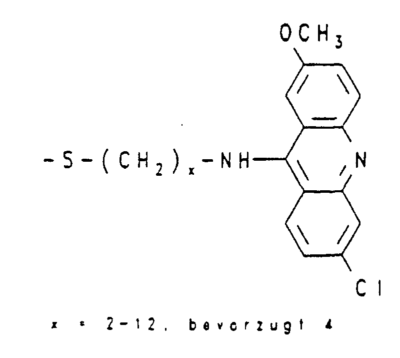

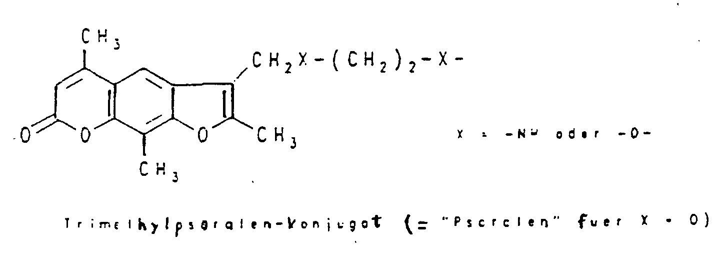

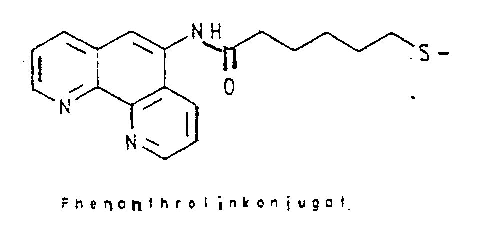

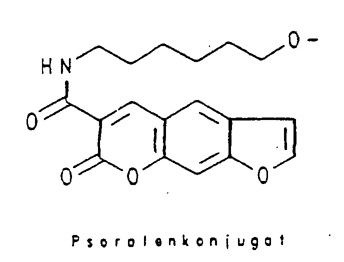

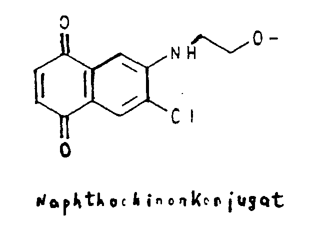

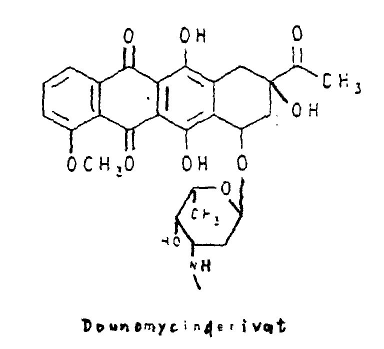

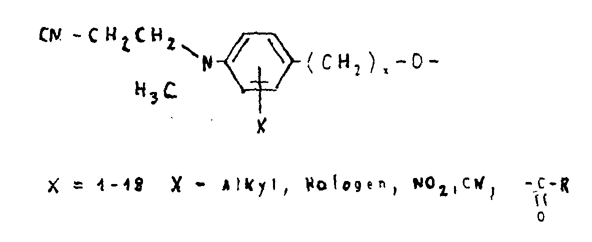

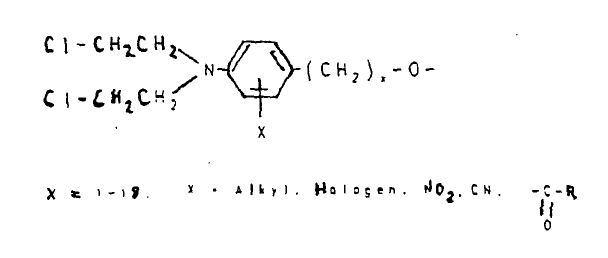

Als Beispiele für Gruppen NR³R⁴, in denen R³ und R⁴ zusammen mit dem sie tragenden Stickstoffatom einen 5- bis 6-gliedrigen heterocyclischen Ring bilden, der zusätzlich ein weiteres Heteroatom enthält, seien der Morpholinyl- und der Imidazolidinyl-Rest genannt.

Die Erfindung ist nicht auf α- und β-D- bzw. L-Ribofuranoside, α- und β-D- bzw. L-Desoxyribofuranoside und entsprechende carbocyclische Fünfringanaloga beschränkt, sondern gilt auch für Oligonucleotidanaloga, die aus anderen Zucker-Bausteinen aufgebaut sind, beispielsweise ringerweiterte und ringverengte Zucker, acyclische oder geeignete andersartige Zucker-Derivate.

Oligonucleotidanaloga der Formel IB besitzen einen 3'5'-Spacer (3'5'S) oder (2'5'S), der die Polarität nicht beeinflußt, aber beispielsweise eine Rückfaltung des Oligonucleotidanalogons oder eien Strangwechsel erlaubt.

Beispiele für S sind Propan-1.3-diol-phosphate, 2-Benzyl- bzw. 2-Octadecyl-oxypropan- 1.3-diolphosphate, Triethylenglykol- oder Hexaethylengykolphosphate, die sich gegebenenfalls auch wiederholen können. Nucleotidanaloga ohne heterozyklische Base und Phenylendialkylen-Reste sind weitere bevorzugte Ausführungsformen von S, insbesondere von (3'3'S). Im allgemeinen ist (5'5'S) aus topologischen Gründen länger als (3'3'S). So besitzt (5'5'S) meistens 20 bis 45, bevorzugt 24 bis 36 unverzweigte Bindungen, während (3'3'S) nur 5 bis 15, vorzugsweise 6 bis 10 unverzweigte Bindungen aufweist, unter der Voraussetzung, daß ein Strangwechsel angestrebt wird. Dient S dagegen zur Rückfaltung des Oligonucleotidanalogons, beispielsweise zur Triplexbildung am Nucleinsäure-Einzelstrang, sind Längen von S von 4 bis 5 Nucleotid-Einheiten von Vorteil. Als Beispiel seien hier Penta-(2-benzyloxy-1,3-propandiol)-hexaphosphat und Hexa-(propan-1.3-diol)-pentaphosphat erwähnt, die jeweils eine (5'5'S)- oder (3'5'S)-Verknüpfung bewirken können.

Die Darstellung von Oligonucleotidanaloga der Formel IB erfolgt ähnlich wie die Synthese biologischer Oligonucleotide in Lösung oder vorzugsweise in fester Phase, gegebenenfalls unter Zuhilfenahme eines automatischen Synthesegeräts.

Die Festphasen-Synthese von Oligonucleotiden mit einem Phosphat- bzw. Phosphatester Rest am 3'-Ende ist nach der Standard-Phosphoramidit Chemie nach Caruthers (M. D. Matteucci and M. H. Caruthers, J. Am. Chem. Soc. 103, 3185 (1981)) nicht möglich, da der erste Nucleotid-Baustein über die 3'-Hydroxy-Gruppe an den festen Träger gebunden ist und daher aus diesen Synthesen stets Oligonucleotide mit einer 3'-Hydroxy-Gruppe resultieren. Es wurden verschiedene Verfahren nach der Festphasen-Methode beschrieben, die aber alle umständlich sind und womit sich oft keine Derivate wie Phosphatester oder Alkylphosphonate herstellen lassen (R. Eritja et al., Tetrahedron Lett. 32, 1511 (1991); P. Kumar et al., Tetrahedron Lett. 32, 967 (1991); W. T. Markiewcz und T.K. Wyrzykiewicz, Phoshorus, Sulfur and Silicon 51/52, 374 (1990); E. Felder et al., Tetrahedron Lett. 25, 3967 (1984); R. Lohrmann and J. Ruth, DNA 3, 122 (1984)).

Gegenstand der Erfindung ist ein Verfahren zur Herstellung der Oligonucleotidanaloga der Formel IB, wobei
a) im ersten Reaktionszyklus eine Nucleotideinheit mit 3'(2')-terminaler Phosphor(V)-Gruppierung und freier 5'-Hydroxy- oder Mercaptogruppe mit einer weiteren Nucleotideinheit mit 3'(2')-ständiger Phosphor(III)- oder Phosphor(V)-Gruppierung oder deren aktiviertem Derivat umgesetzt wird, in den weiteren Zyklen eine Nucleotideinheit mit 3'(2')- bzw. 5'-terminaler Phosphor(III)- oder Phosphor(V)-Gruppierung oder deren aktiviertem Derivat oder mit einem Reagens, das die Einführung von Spacergruppen erlaubt, mit einer weiteren Nucleotideinheit mit 5'- bzw. 3'(2')-terminaler freier Hydroxy- oder Mercaptogruppe umgesetzt wird oder
b) das Oligonucleotidanalogon durch Fragmente in gleicher Weise aufgebaut wird, in den nach (a) oder (b) erhaltenen Oligonucleotidanaloga zum Schutz anderer Funktionen temporär eingeführte Schutzgruppen abgespalten werden und die so erhaltenen Oligonucleotidanaloga der Formel IB gegebenenfalls in ihre physiologisch verträglichen Salze überführt werden.

Als Ausgangskomponente für die Festphasensynthese wird ein fester Träger der Formel IV eingesetzt

D-X'-CH₂CH₂-S(O)ₓ-CH₂CH₂-A-T (IV),

worin
A für einen Linker-Arm steht, der beispielsweise ein Rest einer Dicarbonsäure, eines Diols, eines Alkylamins, eines Dicarbonsäuremonoalkylamids, eines Säureamids oder eines Phosphats der Formel 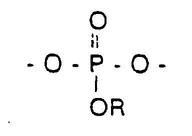 ist, worin R = Wasserstoff oder C₁-C₆-Alkyl, das gegebenenfalls durch -CN substituiert ist, vorzugsweise Methyl oder 2-Cyanoethyl, bedeutet, T ein fester Träger, beispielsweise aus Materialien wie CPG (Controlled Pore Glass), Kieselgel oder einem organischen Harz wie Polystyrol(PS) oder einem Pfropf-Copolymer aus PS und Polyethylenglykol(POE),ist, der durch funktionelle Gruppen wie Hydroxy, Amino, Halogen oder COOH in der Seitenkette modifiziert ist,
D für eine Schutzgruppe steht, die sich ohne Spaltung des Linkerarmes A und des X'-CH₂CH₂-S(O)ₓ-CH₂CH₂-Restes entfernen läßt (siehe Bioorg. Chem. 14 (1986) 274-325) wie 4-Methoxytetrahydropyranoyl und Dimethoxytrityl, vorzugsweise für Dimethoxytrityl, x eine ganze Zahl Null, 1 oder 2 ist und X' Oxy oder Sulfandiyl bedeutet.

Der Linkerarm A, der durch chemische Bindung (Amid, Ester u.a.) den festen Träger T mit dem schwefelhaltigen Rest verbindet (Damka et al., Nucleic Acids Res. 18, 3813 (1990)), ist vorzugsweise ein Bernsteinsäurerest (O-C(O)-CH₂CH₂-C(O)-), ein Oxalsäurerest, (O-C(O)-C(O)-), ein Alkylamin, vorzugsweise LCAA (Long Chain Alkyl Amin), oder Polyethylenglykol. Besonders bevorzugt ist ein Bernsteinsäurerest. In bestimmten Fällen, beispielsweise in Kombination mit Substituenten, die einer längeren Ammoniak-Behandlung nicht standhalten, sind labilere Linker wie der Oxalyl-Linker von Vorteil. Die Herstellung von festen Trägern der Formeln IV a-c ist in Beispiel 1 beschrieben.

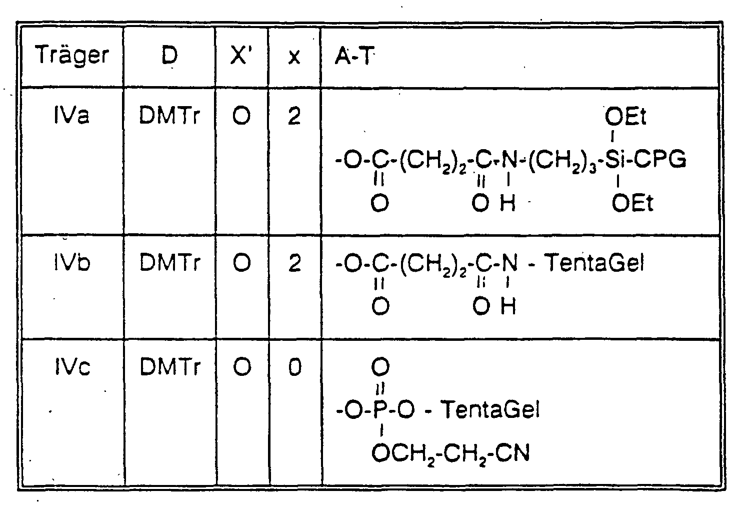

Die Festphasensynthese kann nach der Phosphattriester-Methode, der H-Phosphonat-Methode und der Phosphoramidit-Methode, vorzugsweise nach der Phosphoramidit-Methode (E. Sonveaux, Bioorg. Chem. 14, 274 (1986)), erfolgen. Zunächst wird stets die Schutzgruppe D durch eine Säure, beispielsweise Trichloressigsäure in Methylenchlorid, vom Träger der Formel IV abgespalten. Im Falle der Phosphoramidit-Methode wird der so erhaltene Träger der Formel IV'

HX'-CH₂-CH₂-S(O)ₓ-CH₂CH₂-A-T (IV),

worin x, X', A und T die obengenannte Bedeutung haben, mit einem Nucleosid-Phosphoramidit der Formel V 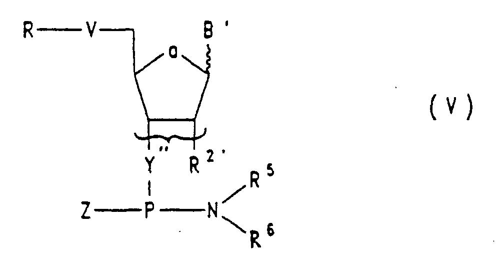 worin
- B': B bedeutet, wobei gegebenenfalls in B vorhandene NH₂-Gruppen geschützt vorliegen können;
- R: eine Schutzgruppe ist, die sich unter milden Bedingungen entfernen läßt wie 4-Methoxytetrahydropyranoyl oder Dimethoxytrityl,
- R²: Wasserstoff, Alkoxy, Halogen oder eine geschützte Hydroxy- oder Aminofunktion ist und
- R⁵ und R⁶: unabhängig voneinander C₁-C₁₂-Alkyl oder beide Reste zusammen einen 5 bis 6-gliedrigen Ring bilden, Y" Oxy, Sulfandiyl oder (CH₂)ₘ ist, und a, m, V und Z die obengenannte Bedeutung haben,
in Gegenwart einer schwachen Säure wie Tetrazol kondensiert. Anschließend oxidiert man den so erhaltenen Träger in an sich bekannter Weise mit Jodwasser (W= O) bzw. mit TETD (Tetraethylthiuramdisulfid) oder elementarem Schwefel, (W=S) bzw. mit Selen (W=Se) zum derivatisierten Träger der Formel VII 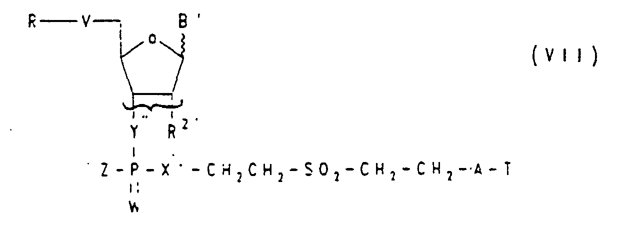 worin
R, V, B', R², Z, X', W, Y", A und T die obengenannte Bedeutung haben. Bevorzugt werden Träger der Formel VIIa 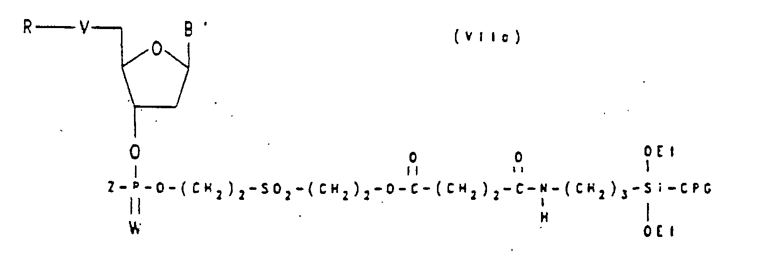 hergestellt.

Das Phosphoramidit der Formel V kann beispielsweise aus dem Bis-Amidit der Formel VI 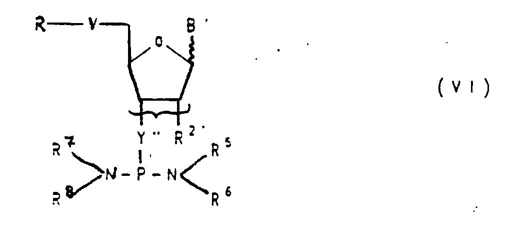 worin
R⁷ und R⁶ gleich R⁵ und R⁶ sind und
a, R, V, B', R², Y", R⁵ und R⁶ die obengenannte Bedeutung haben, durch Umsetzung mit dem entsprechenden Alkohol oder Thioalkohol unter Tetrazol-Katalyse gewonnen werden (Beispiel 2, Methode A), wenn Z = Alkoxy oder Alkylmercapto ist (J. E. Marugg et al., Tedrahedron Lett. 27, 2271 (1986). Bevorzugte Bis-Amidite sind solche der Formel Vla

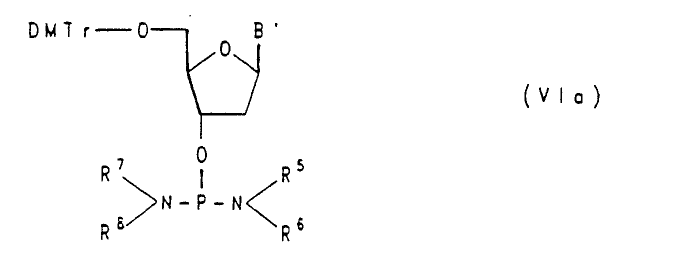

Auf diese Weise wurden beispielsweise die Amidite der Formeln VIII a-m hergestellt, 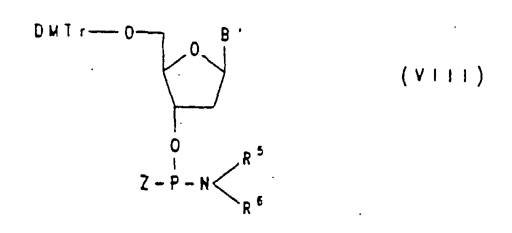 worin
R⁵ und R⁶ die obengenannte Bedeutung haben, Z die Bedeutung hat von
a) O-CH₂CH₃,
b) O-i-C₃H₇,
c) O-n-C₆H₁₃
d) O-n-C₁₈H₃₇,
e)

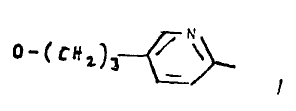

f) 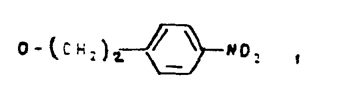
g-k) einem Rest der Formel III, worin bei
g) p = 3 und q = 0,
h) p = 4 und q = 9,
i) p = 5 und q = 4 und bei
k) p = 8 und q = 13 ist,
p) CH₃
m) 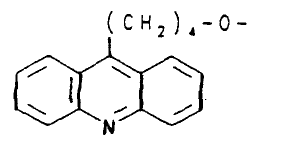
und B' bei
a), c) und d) Cyt^{i-Bu}, bei
b) und p) Thy und bei
e) - k) und m) Cyt^{Bz} bedeutet.

Eine alternative Methode zur Beladung des Trägers ist die Umsetzung des Phosphitylierungsreagens der Formel IX 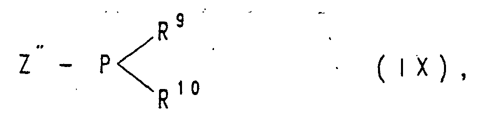 worin
R⁹ und R¹⁰ unabhängig voneinander Cl, NR⁵R⁶, NR⁷R⁸, Z" oder U' bedeuten und wobei U' C₁-C₄-Alkyl oder eine als geschütztes Derivat vorliegende Hydroxygruppe ist, R⁵, R⁶, R⁷ und R⁸ die obengenannte Bedeutung haben, Z" = Z ist, mit der Maßgabe, daß Hydroxy, Mercapto und SeH als geschützte Derivate vorliegen müssen, beispielsweise als Y"'-G'-X'-DMTr, worin DMTr Dimethoxytrityl, X' = Y"' = Oxy oder Sulfandiyl, und G' (CH₂CH₂O)_{α}CH₂CH₂ oder CH₂CH(OR')CH₂ ist mit R'= C₁-C₁₈-Alkyl, C₆-C₁₄-Aryl oder C₆-C₁₄-Aryl-C₁-C₈-alkyl und α = eine ganze Zahl von 1 bis 11, oder als O-CH₂CH₂-CN, O-CH₃, S-CH₂CH₂CN, O-CH₂CH₂-S-CH₂CH₂-O-D
oder 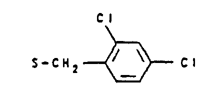 mit einem Nucleosid mit freier 3'(2')-Funktion der Formel X 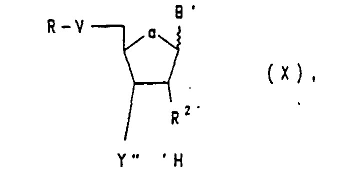 worin
V, B', R^{2'} und R die obengenannte Bedeutung haben und Y"' = Oxy oder Sulfandiyl ist, und anschließende Kondensation der so erhaltenen Verbindung an den Träger der Formel IV' in Gegenwart eines Kondensationsmittels, wie Tetrazol (für R⁹, R¹⁰ = NR⁵R⁶ bzw. NR⁷R⁸) oder Diisopropylamin (für R⁹, R¹⁰ = Cl).

Anschließende Oxidation mit Jodwasser bzw. Schwefel oder Selen führt dann zur Verbindung der Formel Vlla. Nun kann die Schutzgruppe R entfernt werden und die Oligonucleotid-Synthese in bekannter Weise weitergeführt werden. Am Ende der Synthese werden von dem so erhaltenen trägergebundenen Oligonucleotidanalogon in bekannter Weise die Schutzgruppen entfernt und danach wird das erfindungsgemäße Oligonucleotidanalogon der Formel IA oder IB vom Träger abgespalten.

Wurde die Synthese mit einem Baustein der Formel V im letzten Cyclus abgeschlossen, erhält man ein Oligonucleotidanalogon der Formel IA oder IB (R¹ =H) mit einer 5'-Hydroxy-Gruppe und einer phosphorhaltigen Konjugation am 3'(2')-Ende. Setzt man dagegen im letzten Kondensationsschritt ein Phosphorylierungsreagenz, beispielsweise der Formel IX, worin R⁹ = Z" ist, ein, dann resultiert aus der Synthese ein Oligonucleotidanalogon der Formel IA oder IB mit R¹= Formel II, das sowohl am 3'(2')- als auch am 5'-Ende eine phosphathaltige Substitution aufweist.
Die Herstellung von Oligonucleotiden mit einer 3'(2')-terminalen Phosphoramidat-Gruppe ist beispielsweise durch Umsetzung des Trägers der Formel IV' mit dem monomeren Methoxy-Phosphoramidit der Formel V (Z=O-CH₃) 2in Gegenwart von Tetrazol möglich, wenn man die Oxidation wie bei Jäger et al. (Biochemistry 27, 7237 (1988) beschrieben mit Jod/H₂NR³ bzw. HNR³R⁴ ausführt, worin R³ und R⁴ die obengenannte Bedeutung haben.

In bestimmten Fällen (Z=NHR³, NR³R⁴,O, S oder Se) kann die Einführung der Gruppe Z auch nach der H-Phosphonat-Methode erfolgen, indem der zunächst durch Umsetzung eines Nucleosid-Phosphonats der Formel Xl 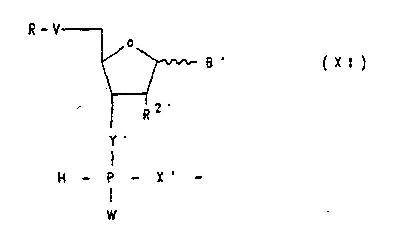 worin R, V, a, B', Y', X' und W die obengenannte Bedeutung haben, mit einem Träger der Formel IV' gebildete H-Phosphonat-Diester der Formel VII' 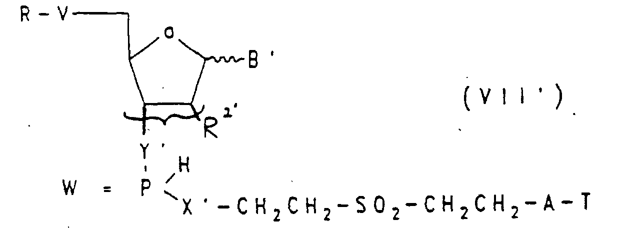 einer oxidativen Phosphoramidierung (B. Froehler, Tetrahedron Lett. 27, 5575 (1986) unterworfen wird. Auf diese Weise läßt sich zum Beispiel mit Cholesteryl-oxycarbonyl-aminoalkylamin in Gegenwart von Tetrachlorkohlenstoff ein Oligonucleotid mit einer 3'-terminalen Cholesteryl-Gruppe herstellen.

Die Herstellung von Oligonucleotidanaloga der Formeln IB ist ferner nach der Triester-Methode möglich, indem man die Gruppe HX' des Trägers der Formel IV' mit einem geschützten Phosphatdiester der Formel XII 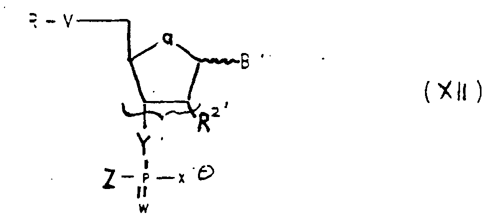 worin R, V, a, B', R², Y', Z, W, X' und die geschweifte Klammer die obengenannte Bedeutung haben, in Gegenwart eines Kondensationsmittels wie Arylsulfonsäurechlorid und eines nucleophilen Katalysators wie Tetrazol umsetzt. Eine Möglichkeit zur Invertierung der Polarität einer in 3'5' (2'5')-Richtung wachsenden Oligonucleotid-Kette besteht darin, daß man die freie 5'-Hydroxy bzw. 5'-Thiolgruppe der wachsenden Kette mit einem Baustein der Formel XIII, beispielsweise mit einem 3'-O-DMTr-Nucleosid-5'-phosphoramidit der Formel Xllla, 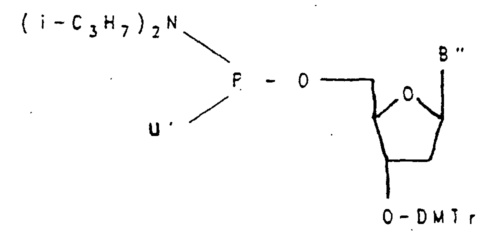 (XIIIa) : U' = OCH₂CH₂CN, B" = B' (XIIIb) : U' = CH₃, B" = H
umsetzt. Oxidiert man anschließend mit Jodwasser bzw. Schwefel, so hat (5'5'S) die Bedeutung eines Phosphat- (PO₄⁻) bzw. eines Phosphorothioat- (PO₃S⁻) Restes. Nach Abspaltung der Schutzgruppe am 3'(2')-Ende kann die Oligonucleotidkette bei Bedarf durch sukzessive Kupplung mit Bausteinen der Formel XIII 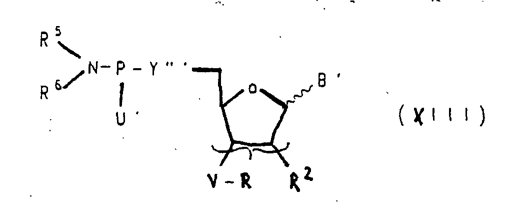 worin
- U': die Bedeutung von U hat, mit der Maßgabe, daß U' nicht NHR³ oder NR³R⁴ bedeutet und Hydroxy, Mercapto und SeH als geschützte Derivate (Beispiele solcher Derivate siehe Z") vorliegen, und
- R⁵, R⁶, Y"', V, R, a, R^{2'} und B': die obengenannte Bedeutung haben,
in 5'3'-Richtung verlängert werden. Die Synthese der Bausteine XIII erfolgt beispielsweise wie in Seliger et al. (Nucleosides, Nucleotides 10 (1991 ), 469) beschrieben.

Zur Herstellung von Oligonucleotidanaloga der Formel IB, deren Polarität unverändert ist, deren (3'5'S) jedoch eine Rückfaltung der Kette erleichtert, wird an gewünschter Stelle die Synthese der Oligonucleotid-Kette mit einem Baustein der Formel XIV verlängert, der aufgrund seiner Bifunktionalität auch mehrfach ankondensiert werden kann.

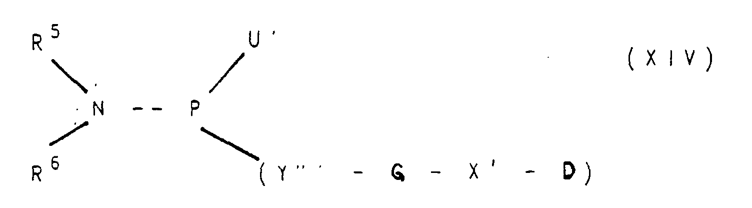

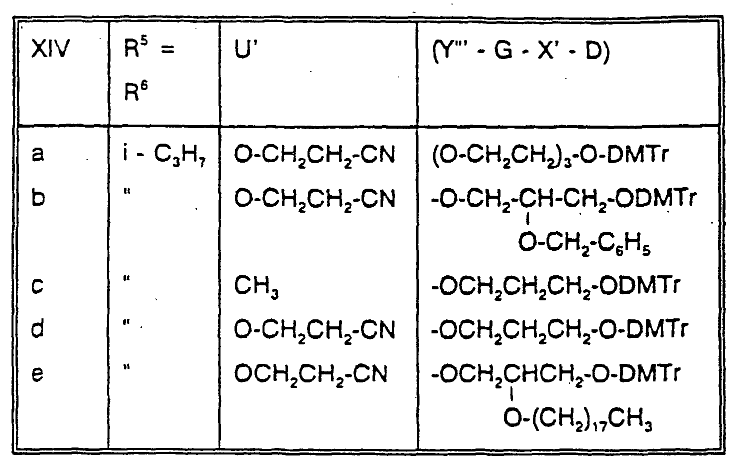

Beispielsweise läßt sich eine (3'5'S)-Schlaufe durch mehrfache, vorzugsweise 4 bis 5-fache sukzessive Kupplung mit dem Baustein der Formel XIVb einführen. Danach wird die Synthese wie vorher beschrieben in 3'5'- Richtung weitergeführt.

Wird bei der Hybridisierung des Oligonucleotidanalogons an doppelsträngige Nucleinsäuren, vorzugsweise DNA, ein Strangwechsel angestrebt, dann muß der 5'5'-Spacer von größerer Länge sein. Beispielsweise kann ein Triethylenglykoldiphosphat an eine in 3'5'-Richtung aufgebaute Kette ein- oder mehrmals, bevorzugt zweimal durch zweimalige Kupplung mit dem Baustein der Formel XIVa an gewünschter Stelle eingebaut werden. Zwecks Änderung der Polarität wird anschließend mit Nucleotid-Bausteinen der Formel XIII die Synthese in 5'3'-Richtung weitergeführt. Ist eine erneute Umkehrung der Polarität erwünscht, so wird an beabsichtigter Stelle ein 3'3'-Spacer eingeführt und anschließend die Synthese der Kette in 3'5'-Richtung durch Kondensation mit Nucleosid-3'-Phosphoramiditen weitergeführt. Als 3'3'-Spacer bietet sich beispielsweise die Einführung einer Propan-1.3-dioldiphosphatgruppe an, die sich durch Kupplung mit dem Baustein der Formel XIVd einführen läßt.

Wird die Oligonucleotid-Synthese mit dem Sulfid- (x=0) oder Sulfinyl- (x=1) Träger durchgeführt, so werden diese Gruppen am Ende in an sich bekannter Weise [Funakoshi et al. Proc. Notl. Acad. Sci. 88 (1991), 6982] zum Sulfonyl-Rest oxidiert, um eine leichte Spaltung mit Basen, vorzugsweise Ammoniak, zu gewähren.

Die Art der Amino-Schutzgruppen der Basen B' und die Beschaffenheit des Linkerarmes A richten sich im Einzelfall nach der Art des Substituenten Z, da diese nach vollständiger Synthese problemlos spaltbar sein müssen. Zum Beispiel kann man bei der Herstellung eines Oligonucleotid-3'-Phosphatisopropylesters (Z = O-i-C₃H₇), für B= Ade und Cyt mit den Benzoyl- (Bz) bzw. für B = Gua mit den Isobutyryl- (i-Bu) Schutzgruppen arbeiten. Dagegen verwendet man zur Synthese eines Oligonucleotid-3'-methylphosphonatesters (Z=CH₃) oder Ethylesters (Z=O-C₂H₅) für B= Ade und Gua bevorzugt die labileren Phenoxyacetyl (PAC) bzw. für B= Cyt die iso-Butyryl-Schutzgruppen.

Manche Konjugate besitzen zusätzliche funktionelle Gruppen, die vor dem Einbau in die monomeren Bausteine der Formel V und XIII in geeigneter Weise geschützt werden müssen. Beispielsweise ist die Carboxyl-Gruppe des Fluoresceins als Alkylester zu schützen. Im Psoralen kann die Amid-Gruppe als N-Fmoc (Fluorenylmethoxycarbonyl) geschützte Verbindung vorliegen. Hydroxygruppen können durch Acylierung oder Silylierung (t-Butyldimethylsilyl) vor Nebenreaktionen geschützt werden. Aminogruppen können auch Trifluoracetyl-geschützt vorliegen. In Ausnahmefällen können die Konjugate so wenig stabil sein, daß sie unter den Bedingungen der Schutzgruppen-Abspaltung der Oligonucleotid-Synthese bereits zerstört würden. In solchen Fällen ist es günstig, im Monomer der Formel V lediglich einen Linker-Arm mit einer funktionellen Gruppe, beispielsweise Z = HN-(CH₂)ₓ-NH-Fmoc, worin x eine ganze Zahl von 2-12, bevorzugt 4-6, bedeutet, einzubauen. Nach Einbau in das Oligonucleotid und Entfernung der Schutzgruppen, vorzugsweise mit Ammoniak, kann die freie Aminogruppe mit Aktivestern gekoppelt werden. So wurde beispielsweise der basenlabile AcridiniumEster hergestellt.

Die Charakterisierung der synthetisierten Oligonucleotid-Derivate erfolgt durch Elektro-Spray-lonisations Massenspektrometrie (Stults u. Masters, Rapid Commun. Mass. Spectr. 5 (1991) 350).

Die erfindungsgemäßen Oligonucleotidanaloga der Formel IB wurden auf ihre Stabilität im Serum und gegenüber bekannten Exonucleasen getestet.

Überraschenderweise wurde gefunden, daß alle Oligonucleotidanaloga der Formel IB im Vergleich zu den nicht modifizierten Oligonucleotiden eine stark erhöhte Stabilität gegenüber den Nucleasen des Serums aufweisen, während ihr Hybridisationsverhalten nur wenig beeinflußt wird.

Während nicht modifizierte Oligonucleotide in fötalem Kälberserum eine Halbwertszeit von etwa zwei Stunden aufweisen, sind alle Oligonucleotidanaloga der Formel IB etwa 16 Stunden ausreichend stabil. Die Oligonucleotidanaloga der Formel I sind darüberhinaus stabil gegenüber Schlangengift-Phosphodiesterase. Nicht modifizierte Oligonucleotide werden von Schlangengift-Phosphodiesterase vom 3'-Ende und von Milz-Phosphodiesterase vom 5'-Ende exonucleolytisch abgebaut.

Die Oligonucleotidanaloga der Formel IB bilden durch Watson-Crick-Basenpaarung mit komplementären einzelsträngigen Nucleotid-Sequenzen stabile doppelsträngige Hybride bzw. durch Watson-Crick und Hoogsteen Basenpaarung stabile Triplex-Strukturen, während sie mit doppelsträngigen Nucleinsäuren über Hoogsteen-Basenpaarung tripelhelicale Strukturen ausbilden, wobei die Spacer (5'5'S) und (3'3'S) einen Strangwechsel ermöglichen. Dadurch ist die Regulation oder Unterdrückung biologischer Funktionen von Nucleinsäuren mit den erfindungsgemäßen Oligonucleotidanaloga möglich, beispielsweise die Unterdrückung der Expression zellulärer Gene wie auch der von Onkogenen oder von viralen Genom-Funktionen. Oligonucleotidanaloga der Formel IB sind daher einsetzbar zur Therapie oder Prophylaxe viraler Infektionen oder Krebskrankheiten.

Die Wirksamkeit der erfindungsgemäßen Oligonucleotide wurde anhand der Inhibition der Virus-Vermehrung von HSV-1 nachgewiesen. 
3' .... GCAGGTACAGCCGTTTGTCGA ... 5'RNA-target
| | | | | | | | | | | | | | | : | : | | |
Sequenz I 3' CGTCCATGTCGGCAAACAGCT 3'
( )
(5'5'S)
 
3' TTGGTGGTGG 5'
Sequenz II 3'....CTCCTGCAAGGAGGACGCCCTTCCG.... 5' -target
TTCCTCCTGCGGGAAGG
(3'5'S). 5' 3'
 
Sequenz III

5' TTGGTGGTGG 3'

3'...CTCCTGCAAGGAGGACGCCCTTCCG ... 5' -target

(5'5'S) TTCCTCCTGCGGGAAGG

5' 3'
 
Sequenz VI
(Ad E1b)    5'    GGGCGGGGCTTAAAGG
                                                    (3'5'S)
            3'    CCCGCCCCGAATTTCC
 
Sequenz IV

3'    5'  3'
|     A - T
G •   G - C
G •   G - C
T •   A - T
G •   C - G
T •   A   -T
T •   A - T
T •   A - T
G •   G - C
G •   T - A
G •   G - C
G •   C - G
G •   G - C
T •   A - T
T •   A - T
G •   C - G
5' G •   G - C
      C - G • G  5'
      T - A • T
      T - A • T
      C - G • G
      G - C • G
      C - G • G
      G - C • G
      T - A • T
      T - A • T
      C - G • G
      T - A • T
      C - G • G
      A - T • T
      C - G • G
      T - A   |
      T - A   3'
      T - A
      T - A
      |   |
      3'  5'

target

HSV-1 (origin)
 
Sequenz V

5' 3' 5'
| | |
T - A ● T
T - A ● T
C - G ● G
T - A ● T
C - G ● G
A - T ● T
T - A ● T
T - A ● T
C - G ● G
T - A ● T
C - G ● G
T - A ● T
T - A ● T
C - G /3'
3'/ C - G
T ● A - T
G ● G - C
T ● A - T
T ● A - T
T ● A - T
G ● C - G
G ● G - C
5' C - G ● G 5'
C - G ● G
T - A ● T
G - C ● G
C - G ● G
C - G ● G
C - G ● G
| | |
3' 5' 3'
target

APP 770
(Alzheimer)
 Die ausgewählte Sequenz I ist in Form der natürlichen Sequenz, also ohne 3'-Derivatisierung und 5'5'-Spacer gegenüber HSV-1 in Zellkultur unwirksam, da sie einem raschen Abbau im Serum unterliegt. Dagegen hemmen 3'-derivatisierte Oligonucleotidanaloga der Formel IA, beispielsweise die Sequenz IA-2 (Beispiel 4b), die Vermehrung von HSV-1. Die Sequenz I stellt nur ein beliebiges Beispiel zur Veranschaulichung des Prinzips dar. Die Sequenz I richtet sich gegen die mRNA des Transaktivator Proteins Vmw65 von HSV-1. Die Sequenzen II und III sind gegen die splice-acceptor Region der IE4/5 Vorläufer-mRNA (IE=immediate early) von HSV-1 gerichtet und hemmen die Replikation von HSV-1 ebenfalls spezifisch. Ein mit Psoralen an beiden 3'-Enden modifiziertes Oligonucleotid der Formel IA-4 (Beispiel 4e) der Sequenz IV erkennt den Replikationsursprung (ori_{L}) des HSV-1 Genoms und hemmt dessen Replikation durch Triplex-Bildung. Die antivirale Wirksamkeit der Psoralen-Konjugate kann durch Bestrahlung mit UV-Licht deutlich gesteigert werden. Das HSV-1 Genom mit seinen 160 000 Basen bietet natürlich unzählig viele andere Zielsequenzen unterschiedlicher Effizienz zur Inhibition der Virus-Vermehrung an. Durch Variation der Nucleotid-Sequenzen läßt sich das therapeutische Prinzip auch auf beliebige andere Viren, Bakterien oder sonstige Pathogene anwenden. Voraussetzung für eine Übertragung auf andere Krankheitsauslöser ist lediglich, daß man die für den Lebenscyclus essentiellen Gene dieser Krankheitserreger kennt. Diese sind in ihrer Sequenz in großer Vielfalt in den sogenannten Gen-Datenbanken niedergelegt. Ähnliches gilt für Onkogene oder andere zelluläre Gene, die in ihrer Funktion unterdrückt werden sollen. Beispiele für andere zelluläre Gene sind solche, die für Enzyme, Rezeptoren, lonenkanäle, Immunmodulatoren, Wachstumsfaktoren oder andere regulatorischen Proteine codieren. Sequenz V richtet sich beispielsweise gegen den APP770 Gen-Promotor, der für die Expression des Vorläufer-Proteins der Plaque-bildenden amyloiden Proteine bei der Alzheimerschen Krankheit verantwortlich gemacht wird. Sequenz VI simuliert als Sense Oligonucleotid die SP1-Bindungsregion des Adenovirus E1b und inhibiert als 3'-modifiziertes Oligonucleotidanalogon mit einem 3'5'-Spacer der Formel IB-2 die Transkription von E1b. Beispiele für Onkogene sind abl, neu, myc, myb, ras, fos, mos, erbB, ets, jun, p53, src und rel.

Sonden für Nucleinsäuren, insbesondere für DNA aus Oligonucleotidanaloga der Formel IA und B bieten gegenüber den literaturbekannten Oligonucleotid-Derivaten mit einer 3'-Hydroxygruppe einerseits den Vorteil erhöhter Nucleasestabilität, andererseits gestatten sie aber auch die Aufnahme gleicher oder unterschiedlicher Marker-Moleküle an beiden Enden des Oligonucleotids. Von Vorteil ist, daß verschiedenene Marker-Gruppierungen innerhalb eines Oligonucleotids selektiv anregen lassen (Doppelmarkierung). Die bifunktionelle Derivatisierung kann auch dazu verwendet werden, um am einen Ende einen Marker und am anderen Ende eine zusätzliche Funktion (beispielsweise Affinitäts-Label) einzuführen. Zu diesem Zweck kann beispielsweise an einem 3'-Ende des Oligonucleotids Biotin eingebaut werden, das Avidin oder Streptavidin erkennt, während am anderen 3'-Ende über einen Alkylamino-Linker ein Acridiniumester Chemilumineszenz-Marker angebracht werden kann.

In vielen Fällen ist zudem das Penetrationsverhalten der erfindungsgemäßen Oligonucleotidanaloga günstiger als bei den nichtmodifizierten Oligonucleotiden, insbesondere wenn lipophile Reste eingeführt wurden. Die erhöhte Serumstabilität der erfindungsgemäßen Oligonucleotid analoga der Formel IB, ihre verbesserte Zellgängigkeit, ihre verbesserte Bindungsaffinität, ihre Fähigkeit, Zielsequenzen durch Modifizierung (Alkylierung, Cross-Linking) selektiv zu zerstören, sowie ihre verbesserte Nachweisbarkeit bei der DNA-Diagnostik, kommen in einer im Vergleich zu den nichtmodifizierten Oligonucleotiden höheren biolgischen Aktivität zum Ausdruck.

Die zuvor genannten diagnostischen, prophylaktischen und therapeutischen Anwendungen der erfindungsgemäßen Oligonucleotidanaloga stellen nur eine Auswahl an repräsentativen Beispielen dar und ihre Verwendung ist daher nicht darauf beschränkt. Darüber hinaus können die erfindungsgemäßen Oligonucleotidanaloga beispielsweise auch als Hilfsmittel in der Biotechnologie und Molekularbiologie eingesetzt werden.

Die Erfindung betrifft weiterhin pharmazeutische Zubereitungen, die eine wirksame Menge einer oder mehrerer Verbindungen der Formel IB oder deren physiologisch verträglichen Salze, gegebenenfalls zusammen mit physiologisch verträglichen Hilfs- und/oder Trägerstoffen und/oder zusammen mit anderen bekannten Wirkstoffen, enthalten sowie ein Verfahren zur Herstellung dieser Zubereitungen, dadurch gekennzeichnet, daß man den Wirkstoff, zusammen mit dem Träger und eventuell weiteren Hilfs-, Zusatz- oder Wirkstoffen in eine geeignete Darreichungsform bringt. Die Applikation erfolgt vorzugsweise intravenös, topisch oder intranasal.

### Beispiel 1: Herstellung eines Trägers der Formel IV

a) Herstellung des Trägers der Formel IVa durch Umsetzung von Aminopropyl-CPG mit dem Succinat des Bishydroxyethylsulfon-dimethoxytritylethers 4.56 g des einfachen Dimethoxytrityl(DMTr)-ethers von Bis-(2-Hydroxyethyl)-sulfon (10 mmol) werden durch zweimaliges Aufnehmen und Einengen in abs. Pyridin getrocknet, in 25 ml abs. Pyridin gelöst, mit 1.78 g (14 mmol) DMAP (Dimethylaminopyridin) und 1.4 g Bernsteinsäureanhydrid (14 mmol) versetzt und diese Mischung 3 Stunden bei Raumtemperatur gerührt. Nach vollständiger Reaktion wird eingeengt, der Rückstand zur Entfernung des Pyridins dreimal in Toluol aufgenommen und eingeengt und danach in 220 ml Methylenchlorid aufgenommen. Die organische Phase wird mit 10 %-iger Zitronensäure (110 ml) und 3-mal mit 110 ml Wasser gewaschen, über Natriumsulfat getrocknet und eingeengt. Der resultierende feste Rückstand wird im Vakuum getrocknet (5.64 g). Von diesem Succinat werden 1.67 g (3 mmol) zweimal in Pyridin abs. aufgenommen und eingeengt und in einer Mischung aus 0.65 ml Pyridin abs. und 6 ml Tetrahydrofuran (THF) abs. gelöst. Dann gibt man eine Lösung aus 420 mg (3 mmol) p-Nitrophenol und 687 mg DCC (Dicyclohexylcarbodiimid, 3.3 mmol) in 2.1 ml THF abs. zu und rührt zwei Stunden bei Raumtemperatur. Nach vollständiger Umsetzung wird vom ausgefallenen Dicyclohexylharnstoff abzentrifugiert. Das Sediment wird in 1 ml abs. Ether aufgeschlämmt und nochmals zentrifugiert. 1.5 g des Aminopropyl-CPG Trägers der Firma Fluka (500 Å, 100 µmol/gr Aminofunktion) werden in einer Mischung aus 1.8 ml DMF abs. und 350 µl Triethylamin aufgeschlämmt, mit den vereinigten vom Sediment abdekantierten Lösungen des Succinat-nitrophenylesters versetzt und 16 Stunden bei Raumtemperatur geschüttelt. Der feste Träger wird abgetrennt und zur Blockierung reaktiver Gruppen mit 3 ml Verkappungsreagenz (Acetanhydrid / 2.6-Lutidin / DMAP; je 0.25 M in THF) eine Stunde bei Raumtemperatur geschüttelt. Dann wird der derivatisierte CPG-Träger abgesaugt, mit Methanol, THF, Methylenchlorid und Ether gewaschen und anschließend im Vakuum bei 40°C getrocknet. Die Beladung des Trägers der Formel IVa mit Dimethoxytrityl-haltiger Komponente beträgt 38 µmol/g.
b) Herstellung des Trägers der Formel IVb durch Umsetzung von TentaGel® (® = eingetragenes Warenzeichen der Fa. Rapp, Tübingen) mit dem Succinat des Bishydroxyethylsulfon-dimethoxytritylethers
   Die Amino-Form des TentaGel-Harzes, einem PS/POE-Copolymer mit 250 µmol/g Aminofunktion (100 mg), werden in einer Mischung aus 360 µl DMF und 70 µl Triethylamin aufgeschlämmt, mit 400 µmol des Succinat-p-nitrophenylesters (Herstellung s. Bsp. 1a) versetzt und 16 Stunden bei Raumtemperatur geschüttelt. Anschließend wird wie in Bsp. 1a) beschrieben aufgearbeitet. Die Beladung des TentaGel-Harzes der Formel IVb mit Dimethoxytrityl-haltiger Komponente beträgt 98 µmol/g.
c) Herstellung des Trägers IVc durch Umsetzung von TentaGel (Hydroxyform) mit dem Phosphitylierungsreagenz der Formel IX (Z" = DMTr-O-CH₂CH₂-S-CH₂CH₂-O-; R⁹ = N(i-C₃H₇)₂; R¹⁰ = O-CH₂CH₂CN).
   Die Hydroxy-Form des TentaGel-Harzes mit 500 µmol/gr Hydroxyfunktion (50 mg) werden mit 10 Äquivalenten des Phosphitylierungsreagenz der Formel IX (Z" = DMTr-O-CH₂CH₂-S-CH₂CH₂-O-, R⁹ = N(i-C₃H₇)₂; R¹⁰ = O-CH₂CH₂CN) in Gegenwart von 25 Äquivalenten Tetrazol in Acetonitril bei 22°C umgesetzt. Nach der Oxidation mit Jodwasser (1,3 g Jod in THF/Wasser/Pyridin; 70:20:5=v:v:v) wird wie in Beispiel 1a aufgearbeitet. Die Beladung des Trägers der Formel IVc mit Dimethoxytrityl-haltiger Komponente beträgt 247 µmol/g.

### Beispiel 2: Herstellung geschützter Nucleosid-3'-Phosphoramidite der Formel VIII

a) Herstellung von VIII a (B'= Cyt^{iBu}, Z=O-CH₂CH₃, R⁵=R⁶=i-C₃H₇)
   2 mmol des Nucleosid-3'-phosphorbisamidits der Formel VI (B'= Cyt^{iBu}, R⁵=R⁵=R⁷=R⁸=i-C₃H₇) werden zweimal in 20 ml Acetonitril abs. aufgenommen und eingesetzt und in 20 ml Acetonitril abs. gelöst. Dann tropft man eine Lösung aus 2.4 mmol Ethanol und 1.2 mmol sublimiertem Tetrazol in 5 ml abs. Acetonitril während 15 Minuten zu. Nachdem noch 2.5 Stunden weitergerührt wurden, verdünnt man mit 75 ml Methylenchlorid und extrahiert die organische Phase mit 50 ml 5 %-iger Natriumhydrogencarbonat-Lösung. Die wässrige Lösung wird 2-mal mit 50 ml Methylenchlorid gewaschen, die vereinigten organischen Phasen über Natriumsulfat getrocknet und im Vakuum konzentriert. Zur Reinigung wird der Rückstand über eine Kieselgelsäule mit Methylenchlorid/n-Heptan/Triethylamin (45:45:10; v:v:v) chromatographiert. Man erhält 0.7 g der gewünschten diastereomeren Substanz als dünnschichtchromatographisch einheitliche Verbindung. (³¹P-NMR σ=146.7, 147.5 ppm). Als Nebenprodukt lassen sich Spuren des entsprechenden Bis-ethyl-phosphits isolieren (³¹P-NMR σ=139.3 ppm).
b) Herstellung von VIII b (B'= Thy, Z=O-i-C₃H₇, R⁵=R⁶=i-C₃H₇)
   Die Herstellung erfolgt durch Phosphitylierung des 5'-O-Dimethoxytrityl-thymidins der Formel X (B'= Thy (β-Stellung); R= DMTr, V = 0, a=0, Y"=0, 2mmol) mit dem Bisamidit der Formel IX (Z" = O-i-C₃H₇, R⁹=R¹⁰=N(i-C₃H₇)₂; 4 mmol) in Gegenwart von Tetrazol (0.5 mmol) in 10 ml abs. Methylenchlorid. Der Ansatz wird wie in Beispiel 2a aufgearbeitet. (³¹P-NMR σ=145.04 ppm, 145.66 ppm)
c) Herstellung von VIII c (B'= Cyt^{iBu}, Z=-O-n-C₆H₁₃, R⁵=R⁶=i-C₃H₇)
   Analog Beispiel 2a aus dem Bisamidit der Formel VIa (B' = Cyt^{iBu}, R⁵=R⁶=R⁷=R⁸=i-C₃H₇) durch Umsetzung mit einem Äquivalent n-Hexanol unter Tetrazol-Katalyse. (³¹P-NMR 148.1 ppm, 148.5 ppm).
d) Herstellung von VIII d (B'= Cyt^{i-Bu}, Z=-O-n-C₁₈H₃₇, R⁵=R⁶=i-C₃H₇)
   Analog Beispiel 2a aus dem Bisamidit der Formel VIa (B' = Cyt^{i-Bu}, R⁵=R⁶=R⁷=R⁸=i-C₃H₇) durch Umsetzung mit einem Äquivalent n-Octadecanol unter Tetrazol-Katalyse. (³¹P-NMR 147.2 ppm, 147.9 ppm).
e) Herstellung von VIII e (B' = Cyt^{Bz}, Z = 3-Pyridylpropan-3-oxy, R⁵=R⁶=R⁷=R⁸=i-C₃H₇).
   Analog Beispiel 2a aus dem Bisamidit der Formel VIa (B' = Cyt^{Bz}, R⁵=R⁶=R⁷=R⁸=i-C₃H₇, R² = H) durch Umsetzung mit einem Äquivalent 3-Pyridin-(propan-3-ol) unter Tetrazol-Katalyse. In diesem Fall konnten die beiden Diastereomeren säulenchromatographisch getrennt werden. (³¹P-NMR Diastereomer 1: 147.7 ppm, Diastereomer 2: 148.2 ppm)
f) Herstellung von VIII f (B'= Cyt^{Bz}, p-Nitrophenylethyl-2-oxy, R⁵=R⁶=i-C₃H₇),
   Analog Beispiel 2a aus dem Bisamidit der Formel VIa (B' = Cyt^{Bz}, R⁵=R⁶=R⁷=R⁸=i-C₃H₇) durch Umsetzung mit einem Äquivalent p-Nitrophenyl-ethan-2-ol unter Tetrazol-Katalyse. (³¹P-NMR 148.1 ppm, 148.6 ppm).
g) Herstellung von VIII g (B'= Cyt^{Bz}, Z=-(OCH₂CH₂)₃OCH₃, R⁵=R⁶=i-C₃H₇)
   Analog Beispiel 2a aus dem Bisamidit der Formel VIa (B' = Cyt^{Bz}, R⁵=R⁶=R⁷=R⁸=i-C₃H₇) durch Umsetzung mit einem Äquivalent Triethylenglycol-monomethylether unter Tetrazol-Katalyse. (³¹P-NMR 148.5 ppm, 148.9 ppm).
h) Herstellung von VIII h (B'= Cyt^{Bz}, Z=-(OCH₂CH₂)₄O(CH₂)₉CH₃, R⁵=R⁶=i-C₃H₇)
   Analog Beispiel 2a aus dem Bisamidit der Formel VIa (B' = Cyt^{Bz}, R⁵=R⁶=R⁷=R⁸=i-C₃H₇) durch Umsetzung mit einem Äquivalent Tetraethylenglycol-monodecylether unter Tetrazol-Katalyse. (³¹P-NMR 148.4 ppm, 148.8 ppm).
i) Herstellung von VIII i (B' = Cyt^{Bz}, Z=-(OCH₂CH₂)₅O(CH₂)₄CH₃, R⁵=R⁶=i-C₃H₇)
   Analog Beispiel 2a aus dem Bisamidit der Formel VIa (B'= Cyt^{Bz}, R⁵=R⁶=R⁷=R⁸=i-C₃H₇) durch Umsetzung mit einem Äquivalent Pentaethylenglycol-monopentylether unter Tetrazol-Katalyse. (³¹P-NMR 148.4 ppm, 148.9 ppm).
k) Herstellung von VIII k (B'= Cyt^{Bz}, Z=-(OCH₂CH₂)₈O(CH₂)₁₃CH₃, R⁵=R⁶=i-C₃H₇)
   Analog Beispiel 2a aus dem Bisamidit der Formel VIa (B' = Cyt^{Bz}, R⁵=R⁶=R⁷=R⁸=i-C₃H₇) durch Umsetzung mit einem Äquivalent Octaethylenglycol-monotetrdecylether unter Tetrazol-Katalyse. (³¹P-NMR 148.4 ppm, 148.8 ppm).
I) Herstellung von VIII p (B'= Thy, Z=CH₃, R⁵=R⁶=i-C₃H₇)
   Analog Beispiel 2b aus 5'-O-Dimethoxytritylthymidin durch Phosphitylierung mit dem Reagenz der Formel IX (Z"=CH₃, R⁹=Cl, R¹⁰=N(i-C₃H₇)₂, wobei anstelle von Tetrazol mit zwei Äquivalenten Diisopropylethylamin katalysiert wird. (³¹P-NMR 120.6 ppm, 121.0 ppm).
m) Herstellung von VIII m (B'= Cyt^{Bz}, Z= Acridin-9-(butyl-4-oxy)-, R⁵=R⁶=i-C₃H₇)
   Analog Beispiel 2a aus dem Bisamidit der Formel VIa (B' = Cyt^{Bz}, R⁵=R⁶=R⁷=R⁸=i-C₃H₇) durch Umsetzung mit einem Äquivalent 9-(4-Hydroxybutyl)acridin unter Tetrazol-Katalyse. (³¹P-NMR 146.7 ppm, 147.4 ppm).

### Beispiel 3: Herstellung des trägergebundenen Nucleotids der Formel VII

a) Methode A: Herstellung eines Trägers der Formel VIIa-1 durch Kopplung des Nucleosid-3'-phosphoramidits der Formel VIIIb 7.5 mg des Trägers aus Beispiel 1a, der 0.2 µmol des Bishydroxyethylsulfon-dimethoxytritylethers gebunden hält, werden mit 3 % Trichloressigsäure behandelt, wobei die DMTr-Schutzgruppe abgespalten wird, mit Acetonitril gewaschen, und anschließend mit 2 µmol des Nucleosid-3'-phosphoramidits der Formel VIIIb (B'= Thy, Z=O-i-C₃H₇, R⁵=R⁶= i-C₃H₇) in Gegenwart von Tetrazol (10 µmol) in Acetonitril zur Reaktion gebracht. Die Reaktionszeit beträgt 2.5 Minuten. Danach wird mit Jod (für W=O; 1.3 g Jod in THF/Wasser/Pyridin; 70:20:5=v:v:v, 5 Minuten) oxidiert.
b) Methode B: Herstellung eines Trägers der Formel Vlla-2 durch Reaktion über das Phosphitylierungsreagenz der Formel IX
   Das Phosphitylierungsreagenz der Formel IX (Z"=n-Octyl, R⁹=R¹⁰=Cl; 1 Äquivalent) wird in Gegenwart von 1.2 Äquivalenten Diisopropylethylamin (DIPEA) in abs. Acetonitril oder Methylenclorid mit einem Nucleosid der Formel X (1 Äquivalent 5'-O-Dimethoxytritylthymidin, B' = β-Stellung, Y"' = 0,) bei -78°C zum entsprechenden Nucleosid-3'-O-n-octylphoshonmonochlorid umgesetzt. Der Träger der Formel IVa wird zur Abspaltung der Schutzgruppe D=DMTr wie in Methode A beschrieben behandelt, mit Acetonitril gewaschen und in Gegenwart von DIPEA mit einem Überschuß des in situ zubereiteten Nucleosid-3'-O-n-octylphoshonmonochlorids umgesetzt. Nach Oxidation mit Jodwasser erhält man ein trägergebundenes Nucleotid der Formel Vlla-2, das für die weitere Oligonucleotid-Synthese zur Verfügung steht.

### Beispiel 4: Herstellung von Oligonucleotiden der Formel IB (Das Monomer ist jeweils ein β-D-Desoxyribonucleosid)

a) Herstellung eines Oligonucleotids der Formel IB-1 (R¹=R²=H, Z=n-C₈CH₁₇, a=U=V=W=X=Y=Y'=O, i=1, d=9, e=16) 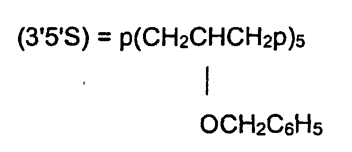 
d(GpGpTpGpGpTpGpGpTpT(3'5'S)TpTpCpCpTpCpCpTpGpCpGpGpGpApApGpGp
-n-C8CH17).
   Ausgehend vom Träger der Formel Vlla-2 (B'=Gua^{PAC}, W=O, Z=n-C₈CH₁₇), dessen Darstellung analog wie in Beispiel 3b beschrieben erfolgt, wird der Kettenaufbau wie in Beispiel 4b der EP-A 0 552 767 durchgeführt. Jedoch verwendet man zur Herstellung basenlabiler Substitutionen (wie hier für Z=n-C₈H₁₇) vorteilhaft die labileren Amino-Schutzgruppen N⁶-Phenoxyacetyl-Ade (Ade^{PAC}), N⁴-Isobutyryl-Cyt (Cyt^{iBu}), N²-Phenoxyacetyl-Gua (Gua^{PAC}), welche am Ende der Synthese leichter zu spalten sind. Nach dem 16. Reaktionszyklus werden 5 Zyklen mit dem Reagenz der Formel XIVb durchgeführt, um den gewünschten (3'5')-Spacer einzuführen. Dann werden die restlichen 10 Nucleotid-Einheiten wie in Beispiel 4a der EP-A 0 552 767 beschrieben eingebaut. Der Spaltung vom Träger (1.5 Stunden Raumtemperatur) mit konz. Ammoniak folgt eine 6-stündige Behandlung mit Ethylendiamin/Ethanol/Wasser (5:4:1; v:v:v) zur Freisetzung der Amino-Funktionen der Basen.
b) Herstellung eines Oligonucleotids der Formel IB-2 (R¹=R²=H, Z=O-(CH₂CH₂O)₈(CH₂)₁₃CH₃, a=U=V=W=X=Y=Y'=O, d=e=15, i=1) (3'5'S) = p(CH₂CH₂CH₂p)₄ 
d(GpGpGpCpGpGpGpGpCpTpTpApApApGpG(3'5'S)CpCpTpTpTpApApGpCpCpCp
CpG pCpCpCp-O-(CH2CH2O)8(CH2)13CH3)
   Ausgehend vom Träger der Formel Vlla-6 (B'=Cyt^{Bz}, W = O, Z=-O-(CH₂CH₂O)₈(CH₂)₁₃CH₃), der wie in Beispiel 3a beschrieben mit Hilfe des Amidits der Formel VIIIk hergestellt wurde, erfolgt die Oligonucleotid-Synthese zunächst analog Beispiel 4b der EP-A 0 552 767. Nach dem 15. Reaktionszyklus werden 4 Zyklen mit dem Baustein der Formel XIVd durchgeführt. Die restlichen 16 Nucleotide werden wie in Beispiel 4b mit 5'-O-DMTr-Nucleosid-3'-phosphoramiditen eingebaut.

### Beispiel 5: Überprüfung auf Nuclease-Stabilität

10 nmol des zu untersuchenden Oligonucleotids werden in 450 µl 20 %-igem fötalem Kälberserum in RPMI-Medium und 50 ml bidestilliertem Wasser gelöst und bei 37°C inkubiert. Dann werden sofort und nach 1, 2, 4, 7 und 24 Stunden 10 µl Proben für die Gelelektrophorese bzw. 20 µl Proben für die HPLC entnommen, zum Abbruch der Reaktion mit 5 µl bzw. 10 µl Formamid versetzt und 5 Minuten auf 95°C erhitzt. Für die Gelelektrophorese werden die Proben auf ein 15 % Polyacrylamid-Gel (2 % BIS) aufgetragen und dieses bei etwa 3000 Voltstunden entwickelt. Die Banden werden durch die Silberfärbung sichtbar gemacht. Zur HPLC Analyse werden die Proben auf eine Gen-Pak Fax HPLC Säule (Fa. Waters/Millipore) gespritzt und bei 1 ml/min mit 5 bis 50 % Puffer A in B chromatographiert (Puffer A: 10mM Natrium-dihydrogenphosphat, 0.1M NaCI in Acetonitril/Wasser 1:4 (v:v) pH 6.8; Puffer B: wie A, jedoch 1.5 M NaCl).

### Beispiel 6: Antivirale Aktivität

Die antivirale Wirkung der erfindungsgemäßen Verbindungen wird in in vitro Versuchen geprüft. Dazu werden die erfindungsgemäßen Verbindungen in verschiedenen Verdünnungen zu Zellkulturen von HeLa- und Verozellen in Mikrotiterplatten gegeben. Nach 3 h werden die Kulturen mit verschiedenen humanpathogenen Viren (z.B: Herpesviren HSV-1, HSV-2, Orthomyxoviren Influenza A2, Picornaviren Rhinovirus 2) infiziert. 48 bis 72 h nach der Infektion wird der Therapieerfolg anhand des cytopathogenen Effektes mikroskopisch und nach Neutralrot-Aufnahme (Farbtest nach Finter) photometrisch bestimmt (Finter, N.B., In "Interferones", N.B. Finter et al., North Holland Publishing Co., Amsterdam, 1966). Die minimale Konzentration, bei der etwa die Hälfte der infizierten Zellen keinen cytopathogenen Effekt zeigen, wird als minimale Hemmkonzentration (MHK) betrachtet.

## Patentansprüche

1. Oligonucleotidanaloga der Formel IB 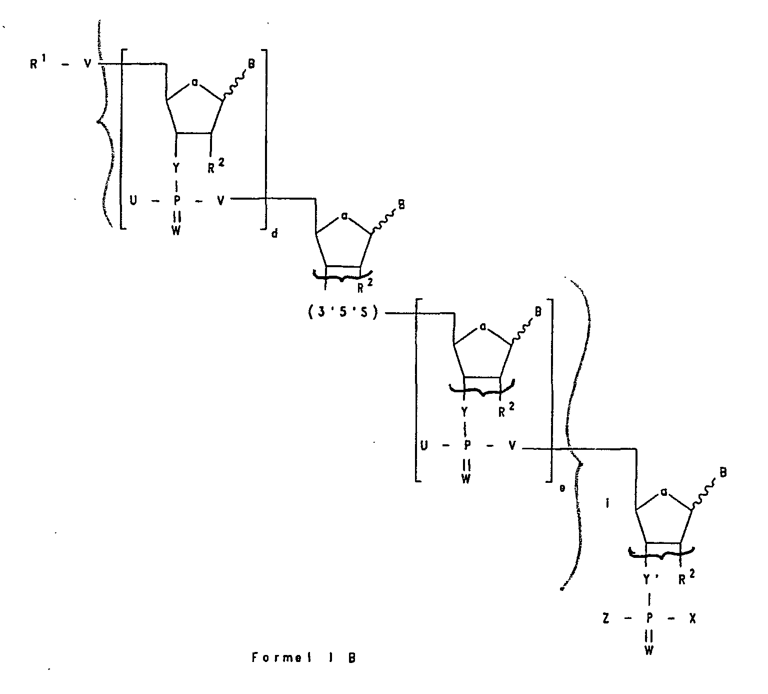 sowie deren physiologisch verträglichen Salze, **dadurch gekennzeichnet, daß**
R¹ Wasserstoff, C₁-C₁₈-Alkyl, C₂-C₁₈-Alkenyl, C₂-C₁₈- Alkinyl, C₂-C₁₈-Alkylcarbony C₃-C₁₉-Alkenylcarbonyl, C₃-C₁₉-Alkinylcarbonyl, C₆-C₂₀-Aryl, (C₆-C₁₄)-Aryl-(C₁-C₈)-alkyl, oder einen Rest der Formel II 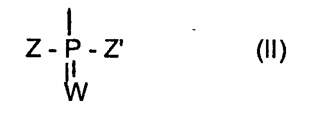
bedeutet;
R² Wasserstoff, Hydroxy, C₁-C₁₈-Alkoxy, Halogen, Azido oder NH₂ bedeutet;
B für eine in der Nucleotidchemie übliche Base steht;
a für Oxy oder Methylen steht;
d,e unabhängig voneinander eine ganze Zahl von 0 bis 50 bedeuten;
i eine ganze Zahl von 1 bis 10 ist;
W Oxo, Selenoxo oder Thioxo bedeutet;
V Oxy, Sulfandiyl oder Imino bedeutet;
Y Oxy, Sulfandiyl, Imino oder Methylen bedeutet;
Y' Oxy, Sulfandiyl, Imino, (CH₂)ₘ oder V(CH₂)ₘ bedeutet, worin
m eine ganze Zahl von 1 bis 18 bedeutet;
X Hydroxy oder Mercapto bedeutet;
U Hydroxy, Mercapto, SeH, C₁-C₁₈-Alkoxy, C₁-C₁₈-Alkyl, C₆-C₂₀-Aryl, (C₆-C₁₄)-Aryl-(C₁-C₈)-alkyl, NHR³, NR³R⁴ oder einen Rest der Formel (OCH₂CH₂)ₚO(CH₂)_{q}CH₂R¹¹ bedeutet, worin
R³ C₁-C₁₈-Alkyl, vorzugsweise C₁-C₈-Alkyl, C₆-C₂₀-Aryl, (C₆-C₁₄)-Aryl-(C₁-C₈)-alkyl, -(CH₂)_{c}-[NH(CH₂)_{c}]_{d}-NR¹²R¹², worin c eine ganze Zahl von 2 bis 6 und d eine ganze Zahl von 0 bis 6 ist, und R¹² unabhängig voneinander Wasserstoff oder C₁-C₆-Alkyl oder C₁-C₄-Alkoxy-C₁-C₆-alkyl ist;
R⁴ C₁-C₁₈-Alkyl, C₆-C₂₀-Aryl oder (C₆-C₁₀)-Aryl-(C₁-C₈)-alkyl bedeutet oder im Falle von NR³R⁴ zusammen mit R³ und dem sie tragenden Stickstoffatom einen 5-6-gliedrigen heterozyklischen Ring bedeutet, der zusätzlich ein weiteres Heteroatom aus der Reihe O, S, N enthalten kann,
p eine ganze Zahl von 1 bis 100 ist,
q eine ganze Zahl von 0 bis 18 ist,
R¹¹ Wasserstoff oder eine funktionelle Gruppe Hydroxy, Amino, NHR¹², COOH, CONH₂, COOR¹² oder Halogen bedeutet, worin R¹² C₁-C₄-Alkyl bedeutet;
S eine Gruppe der Formel III ist 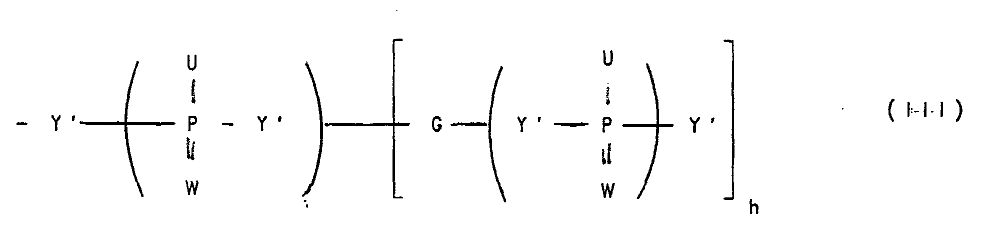
worin
h vier oder fünf ist;
G die Bedeutung hat von C₁-C₁₂-Alkylen, wobei Alkylen gegebenenfalls durch Halogen, Amino, Hydroxy, C₁-C₁₈-Alkyl, C₁-C₁₈-Alkoxy, C₁-C₁₈-Alkylcarbonyloxy, C₆-C₁₄-Aryl, C₆-C₁₄-Aryl-C₁-C₁₈-alkyl, oder C₆-C₁₄-Aryl-C₁-C₈-alkoxy substituiert sein kann, C₆-C₁₄-Aryl-di-C₁-C₈-alkylen, C₆-C₁₈-Arylen einer Gruppe der Formel (CH₂CH₂V)_{*α*} CH₂CH₂ oder (CH₂V)_{α}CH₂, worin α eine ganze Zahl von 1 bis 11 ist,
einer Einheit der Formel 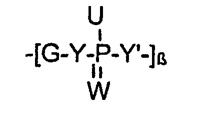
worin β eine ganze Zahl von 1 bis 6 bedeutet, oder einer Gruppe der Formel 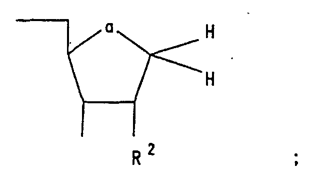
Z=Z' Hydroxy, Mercapto, SeH, C₁-C₂₂-Alkoxy, -O-(CH₂)_{b}-NR¹²R¹³, worin b eine ganze Zahl von 1 bis 6 ist, und R¹³ C₁-C₆₋ Alkyl ist oder R¹² und R¹³ zusammen mit dem sie tragenden Stickstoffatom einen 3-6 gliedrigen Ring bilden, C₁-C₁₈-Alkyl C₁-C₈-Alkyl, C₆-C₂₀-Aryl, (C₆-C₁₄)-Aryl-(C₁-C₈)-alkyl, (C₆-C₁₄)-Aryl-(C₁-C₈)-alkoxy, wobei Aryl auch Heteroaryl bedeutet und Aryl gegebenenfalls durch 1, 2 oder 3 gleiche oder verschiedene Reste aus der Reihe Carboxy, Amino, Nitro, C₁-C₄-Alkylamino, Hydroxy, Halogen und Cyano substituiert ist, C₁-C₁₈-Alkylmercapto, NHR³, NR³R⁴, einen Rest der Formel III oder eine Gruppe bedeuten, die die intrazelluläre Aufnahme begünstigt oder als Markierung einer DNA-Sonde dient;
die geschweifte Klammer in 2'- und 3'-Stellung andeutet, daß sich R² und der benachbarte Phosphorylrest auch umgekehrt in 3'- und 2'-Stellung befinden können,
wobei jedes Nucleotid in seiner D- bzw. L-Konfiguration vorliegen kann und sich die Base B in α- bzw. β-Stellung befinden kann.

2. Oligonucleotidanaloga gemäß Anspruch 1, **dadurch gekennzeichnet, daß** sich die Base B in β-Stellung befindet, die Nucleotide in der D-Konfiguration vorliegen, R² sich in 2'-Stellung befindet und a für Oxy steht.

3. Oligonucleotidanaloga gemäß Anspruch 1 oder 2, **dadurch gekennzeichnet, daß**
R¹ Wasserstoff, C₁-C₆-Alkyl, insbesondere Methyl, oder einen Rest der Formel II bedeutet;
R² Wasserstoff oder Hydroxy, insbesondere Wasserstoff, bedeutet;
d, e eine ganze Zahl von 5 bis 15 bedeuten;
i eine ganze Zahl von 1 bis 3 bedeutet;
m eine ganze Zahl von 1 bis 6, insbesondere 1, bedeutet;
U Hydroxy, Mercapto, C₁-C₆-Alkoxy, C₁-C₆-Alkyl, NR³R⁴ oder NHR³, insbesondere Hydroxy oder C₁-C₆-Alkyl, bedeutet, worin
R³ C₁-C₈-Alkyl, bevorzugt C₁-C₄-Alkyl, oder Methoxyethyl ist, und B, W, V, Y, Y', X und Z die obengenannte Bedeutung haben.

4. Oligonucleotidanaloga gemäß einem oder mehreren der Ansprüche 1, 2 oder 3, **dadurch gekennzeichnet, daß** V, Y und Y' die Bedeutung von Oxy haben.

5. Oligonucleotidanaloga gemäß einem oder mehreren der Ansprüche 1-3 oder 4, **dadurch gekennzeichnet, daß** W die Bedeutung von Oxo hat.

6. Oligonucleotidanaloga gemäß einem oder mehreren der Ansprüche 1-4 oder 5, **dadurch gekennzeichnet, daß** U die Bedeutung von Hydroxy hat.

7. Oligonucleotidanaloga gemäß einem oder mehreren der Ansprüche 1-4 oder 5, **dadurch gekennzeichnet, daß** R¹ für Wasserstoff steht.

8. Verfahren zur Herstellung von Oligonucleotidanaloga der IB gemäß Anspruch 1, wobei
a) im ersten Reaktionszyklus eine Nucleotideinheit mit 3'(2')-terminaler Phosphor(V)-Gruppierung und freier 5'-Hydroxy- oder Mercaptogruppe mit einer weiteren Nucleotideinheit mit 3'(2')-ständiger Phosphor(III)- oder Phosphor(V)-Gruppierung oder deren aktiviertem Derivat oder mit einem Reagens, das die Einführung von Spacergruppen erlaubt, umgesetzt wird, in den weiteren Zyklen eine Nucleotideinheit mit 3'(2')-bzw. 5'-terminaler Phosphor(III)- oder Phosphor(V)-Gruppierung oder deren aktiviertem Derivat einer weiteren Nucleotideinheit mit 5'- bzw. 3'(2')-terminaler freier Hydroxy- oder Mercaptogruppe umgesetzt wird oder
b) die Oligonucleotidanaloga durch Fragmente in gleicher Weise aufgebaut werden,
in den nach (a) oder (b) erhaltenen Oligonucleotiden zum Schutz anderer Funktionen temporär eingeführte Schutzgruppen abgespalten werden und die so erhaltenen Oligonucleotidanaloga der Formel IB gegebenenfalls in ihre physiologisch verträglichen Salze überführt werden.

9. Verwendung der Oligonucleotidanaloga gemäß einem oder mehreren- der Ansprüche 1-6 oder 7 zur Herstellung eines Arzneimittels für die Inhibition der Genexpression.

10. Verwendung der Oligonucleotidanaloga gemäß einem oder mehreren der Ansprüche 1-6 oder 7 zur Herstellung eines Sonden zum Nachweis von Nucleinsäuren.

11. Pharmazeutische Zubereitung enthaltend eih oder mehrere Oligonucleotidanaloga der Formel IB gemäß einem oder mehreren der Ansprüche 1-6 oder 7, gegebenenfalls zusammen mit physiologisch verträglichen Hilfs- und/oder Trägerstoffen.

## Claims

1. Oligonucleotide analogues of the formula IB 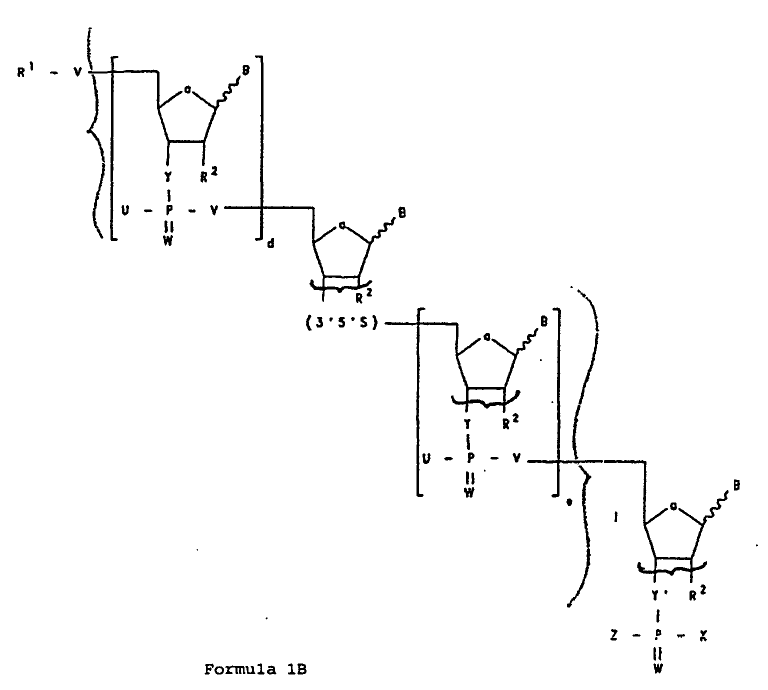 and their physiologically tolerated salts, **characterized in that**
R¹ is hydrogen, C₁-C₁₈-alkyl, C₂-C₁₈-alkenyl, C₂-C₁₈-alkynyl, C₂-C₁₈-alkylcarbonyl, C₃-C₁₉-alkenylcarbonyl, C₃-C₁₉-alkynylcarbonyl, C₆-C₂₀-aryl, (C₆-C₁₄)-aryl-(C₁-C₈)-alkyl, or a radical of the formula II 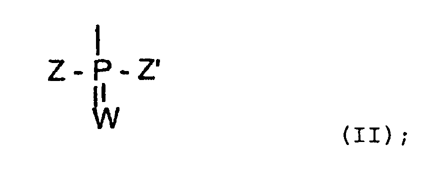
R² is hydrogen, hydroxyl, C₁-C₁₈-alkoxy, halogen, azido or NH₂;
B is a conventional base in nucleotide chemistry;
a is oxy or methylene;
d and e independently of each other are an integer from 0 to 50;
i is an integer from 1 to 10;
W is oxo, selenoxo or thioxo;
V is oxy, sulphanediyl or imino;
Y is oxy, sulphanediyl, imino or methylene;
Y' is oxy, sulphanediyl, imino, (CH₂)ₘ or V(CH₂)ₘ, where
m is an integer from 1 to 18;
X is hydroxyl or mercapto;
U is hydroxyl, mercapto, SeH, C₁-C₁₈-alkoxy, C₁-C₁₈-alkyl, C₆-C₂₀-aryl, (C₆-C₁₄)-aryl-(C₁-C₈)-alkyl, NHR³, NR³R⁴ or a radical of the formula (OCH₂CH₂)ₚO(CH₂)_{q}CH₂R¹¹, where
R³ is C₁-C₁₈-alkyl, preferably C₁-C₈-alkyl, C₆-C₂₀-aryl, (C₆-C₁₄)-aryl-(C₁-C₈)-alkyl, -(CH₂)_{c}-[NH(CH₂)_{c}]_{d}-NR¹²R¹², where c is an integer from 2 to 6 and d is an integer from 0 to 6, and each R¹² independently of the other is hydrogen or C₁-C₆-alkyl or C₁-C₄-alkoxy-C₁-C₆-alkyl;
R⁴ is C₁-C₁₈-alkyl, C₆-C₂₀-aryl or (C₆-C₁₀)-aryl-(C₁-C₈)-alkyl, or, in the case of NR³R⁴, is, together with R³ and the nitrogen atom carrying them, a 5-6-membered heterocyclic ring, which can additionally contain a further hetero atom selected from the group comprising O, S, N,
p is an integer from 1 to 100,
q is an integer from 0 to 18,
R¹¹ is hydrogen or a functional group > hydroxyl, amino, NHR¹²; COOH, CONH₂, COOR¹² or halogen, in which R¹² is C₁-C₄-alkyl;
S is a group of the formula III 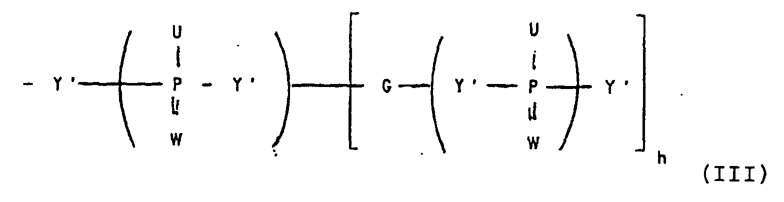
where
h is four or five;
G has the meaning C₁-C₁₂-alkylene, where alkylene can optionally be substituted by halogen, amino, hydroxyl, C₁-C₁₈-alkyl, C₁-C₁₈-alkoxy, C₁-C₁₈-alkylcarbonyloxy, C₆-C₁₄-aryl, C₆-C₁₄-aryl-C₁-C₁₈-alkyl, or C₆-C₁₄-aryl-C₁-C₈-alkoxy, C₆-C₁₄-aryl-di-C₁-C₈-alkylene, C₆-C₁₈-arylene, a group of the formula (CH₂CH₂V)_{α}CH₂CH₂ or (CH₂V)_{α}CH₂, where α is an integer from 1 to 11,
a unit of the formula 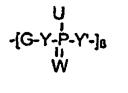 where β is an integer from 1 to 6, or a group of the formula 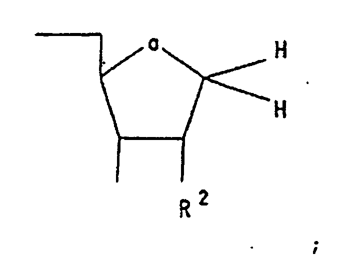
Z = Z' are hydroxyl, mercapto, SeH, C₁-C₂₂-alkoxy, -O-(CH₂)_{b}-NR¹²R¹³, where b is an integer from 1 to 6, and R¹³ is C₁-C₆-alkyl or R¹² and R¹³ together with the nitrogen atom carrying them form a 3-6-membered ring, C₁-C₁₈-alkylC₁-C₈-alkyl, C₆-C₂₀-aryl, (C₆-C₁₄)-aryl-(C₁-C₈)-alkyl, (C₆-C₁₄)-aryl-(C₁-C₈)-alkoxy, where aryl includes heteroaryl, and aryl is optionally substituted by 1, 2 or 3 identical or different radicals selected from the group comprising carboxyl, amino, nitro, C₁-C₄-alkylamino, hydroxyl, halogen and cyano, C₁-C₁₈-alkylmercapto, NHR³, NR³R⁴, a radical of the formula III or a group which favours intracellular uptake or serves as the label for a DNA probe;
the curved bracket in the 2'- and 3'-position indicates that R² and the neighbouring phosphoryl residue can also be located the opposite way round in the 3'- and 2'-position,
where each nucleotide can be present in its D- or L-configuration and the base B can be located in the α- or β-position.

2. Oligonucleotide analogues according to Claim 1, **characterized in that** the base B is located in the β-position, the nucleotides are present in the D-configuration, R² is located in the 2'-position and a is oxy.

3. Oligonucleotide analogues according to Claim 1 or 2, **characterized in that**
R¹ is hydrogen, C₁-C₆-alkyl, in particular methyl, or a radical of the formula II;
R² is hydrogen or hydroxyl, in particular hydrogen;
d and e are an integer from 5 to 15;
i is an integer from 1 to 3;
m is an integer from 1 to 6, in particular 1;
U is hydroxyl, mercapto, C₁-C₆-alkoxy, C₁-C₆-alkyl, NR³R⁴ or NHR³, in particular hydroxyl or C₁-C₆-alkyl, where
R³ is C₁-C₈-alkyl, preferably C₁-C₄-alkyl, or methoxyethyl, and B, W, V, Y, Y', X and Z have the abovementioned meaning.

4. Oligonucleotide analogues according to one or more of Claims 1, 2 or 3, **characterized in that** V, Y and Y' have the meaning of oxy.

5. Oligonucleotide analogues according to one or more of Claims 1-3 or 4, **characterized in that** W has the meaning of oxo.

6. Oligonucleotide analogues according to one or more of Claims 1-4 or 5, **characterized in that** U has the meaning of hydroxyl.

7. Oligonucleotide analogues according to one or more of Claims 1-4 or 5, **characterized in that** R¹ is hydrogen.

8. Process for preparing oligonucleotide analogues of IB according to Claim 1, in which
a) in the first reaction cycle a nucleotide unit with a 3'(2')-terminal phosphorus(V) grouping and a free 5'-hydroxyl or mercapto group is reacted with a further nucleotide unit with in the 3'(2') position a phosphorus(III) or phosphorus(V) grouping or its activated derivative, or with a reagent which permits the introduction of spacer groups, and in the subsequent cycles a nucleotide unit with a 3'(2')- or 5'-terminal phosphorus(III) or phosphorus(V) grouping, or its activated derivative, is reacted with a further nucleotide unit with a 5'- or 3'(2')-terminal free hydroxyl or mercapto group or
b) the oligonucleotide analogues are constructed with fragments in a similar manner,
and protective groups, which have been temporarily introduced in the oligonucleotides obtained according to (a) or (b) in order to protect other functions, are removed and the oligonucleotide analogues of the formula IB thus obtained are, where appropriate, converted into their physiologically tolerated salts.

9. Use of the oligonucleotide analogues according to one or more of Claims 1-6 or 7 for the production of a drug for the inhibition of gene expression.

10. Use of the oligonucleotide analogues according to one or more of Claims 1-6 or 7 for the production of a probe for detecting nucleic acids.

11. Pharmaceutical preparation containing one or more oligonucleotide analogues of the formula IB according to one or more of Claims 1-6 or 7, where appropriate together with physiologically tolerated adjuvants and/or excipients.

## Revendications

1. Analogues d'oligonucléotides de formule IB 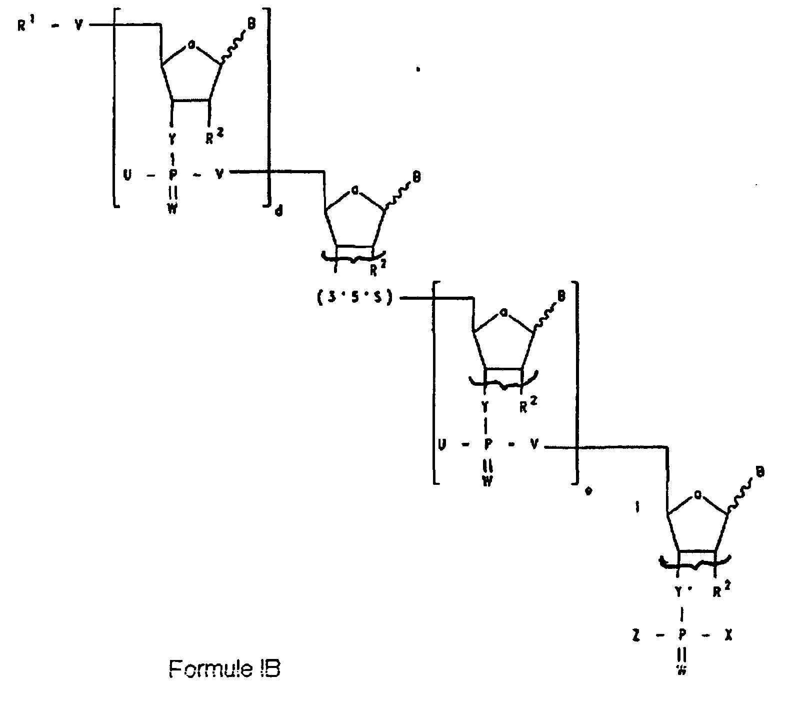 ainsi que leurs sels physiologiquement compatibles, **caractérisés en ce que** R¹ signifie hydrogène, alkyle en C₁ à C₁₈, alcényle en C₂ à C₁₈, alcynyle en C₂ à C₁₈, alkylcarbonyle en C₂ à C₁₈, alcénylcarbonyle en C₃ à C₁₉, alcynylcarbonyle en C₃ à C₁₉, aryle en C₆ à C₂₀, (aryle en C₆ à C₁₄) (alkyle en C₁ à C₈) ou un radical de formule II 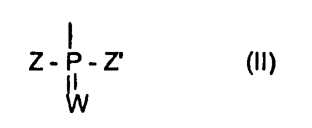 R² signifie hydrogène, hydroxy, alcoxy en C₁ à C₁₈, halogène, azido ou NH₂ ;
B représente une base usuelle dans la chimie des nucléotides ;
a représente oxy ou méthylène ;
d,e signifient, indépendamment l'un de l'autre, un nombre entier de 0 à 50 ;
i représente un nombre entier de 1 à 10 ;
W signifie oxo, sélénoxo ou thioxo ;
V signifie oxy, sulfanediyle ou imino ;
Y signifie oxy, sulfanediyle, imino ou méthylène ;
Y' signifie oxy, sulfanediyle, imino, (CH₂)ₘ ou V (CH₂)ₘ, où
m signifie un nombre entier de 1 à 18 ;
X signifie hydroxy ou mercapto ;
U signifie hydroxy, mercapto, SeH, alcoxy en C₁ à C₁₈, alkyle en C₁ à C₁₈, aryle en C₆ à C₂₀, (aryle en C₆ à C₁₄)(alkyle en C₁ à C₈), NHR³, NR³R⁴ ou un radical de formule (OCH₂CH₂)ₚO(CH₂)_{q}CH₂R¹¹, où
R³ signifie alkyle en C₁ à C₁₈, de préférence alkyle en C₁ à C₈, aryle en C₆ à C₂₀, (aryle en C₆ à C₁₄) (alkyle en C₁ à C₈), -(CH₂)_{c}-[NH(CH₂)_{c}]_{d}-NR¹²R¹², où c est un nombre entier de 2 à 6 et d un nombre entier de 0 à 6, et R¹² représente, indépendamment l'un de l'autre, hydrogène, alkyle en C₁ à C₆ ou (alcoxy en C₁ à C₄)(alkyle en C₁ à C₆) ;
R⁴ signifie alkyle en C₁ à C₁₈, aryle en C₆ à C₂₀ ou (aryle en C₆ à C₁₀) (alkyle en C₁ à C₈) ou, dans le cas de NR³R⁴, ensemble avec R³ et l'atome d'azote qui les porte, un hétérocycle à 5-6 chaînons, qui peut contenir en outre un autre hétéroatome de la série O, S, N ;
p représente un nombre entier de 1 à 100 ;
q représente un nombre entier de 0 à 18 ;
R¹¹ signifie hydrogène ou un groupe fonctionnel hydroxy, amino, NHR¹², COOH, CONH₂, COOR¹² ou halogène où R¹² signifie alkyle en C₁ à C₄ ;
S représente un groupe de formule III 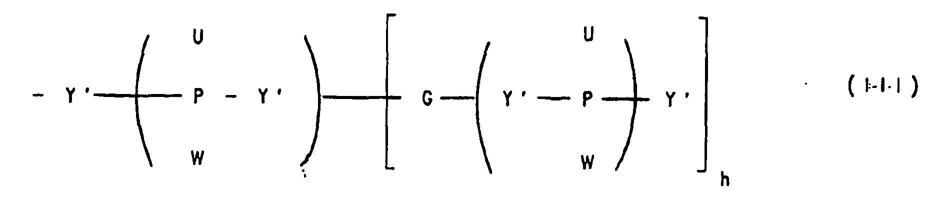 dans laquelle
h représente quatre ou cinq ;
G signifie alkylène en C₁ à C₁₂, alkylène pouvant le cas échéant être substitué par halogène, amino, hydroxy, alkyle en C₁ à C₁₈, alcoxy en C₁ à C₁₈, (alkyle en C₁ à C₁₈)carbonyloxy, aryle en C₆ à C₁₄, (aryle en C₆ à C₁₄) (alkyle en C₁ à C₁₈) ou (aryle en C₆ à C₁₄) (alcoxy en C₁ à C₈), ou signifie (aryle en C₆ à C₁₄)di(alkylène en C₁ à C₈), arylène en C₆ à C₁₈, un groupe de formule (CH₂CH₂V)_{α}CH₂CH₂ ou (CH₂V)_{α}CH₂, où α est un nombre entier de 1 à 11, une unité de formule 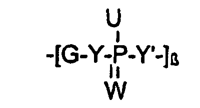 dans laquelle β est un nombre entier de 1 à 6, ou un groupe de formule 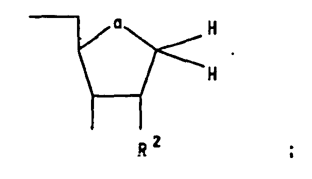 Z = Z' signifient hydroxy, mercapto, SeH, alcoxy en C₁ à C₂₂, -O- (CH₂)_{b}-NR¹²R¹³, où b représente un nombre entier de 1 à 6, et R¹³ représente alkyle en C₁ à C₆ ou R¹² et R¹³ représentent, ensemble avec l'atome d'azote qui les porte, un cycle à 3-6 chaînons, alkyle en C₁ à C₁₈ alkyle en C₁ à C₈, aryle en C₆ à C₂₀, (aryle en C₆ à C₁₄) (alkyle en C₁ à C₈), (aryle en C₆ à C₁₄) (alcoxy en C₁ à C₈), où aryle signifie également hétéroaryle et aryle est le cas échéant substitué par 1, 2 ou 3 radicaux identiques ou différents de la série carboxy, amino, nitro, (alkyle en C₁ à C₄)amino, hydroxy, halogène et cyano, (alkyle en C₁ à C₁₈)mercapto, NHR³, NR³R⁴, un radical de formule III ou un groupe qui favorise l'absorption intracellulaire ou qui sert de sonde d'ADN ;
l'accolade en position 2' et 3' indique que R² et le radical phosphoryle adjacent peuvent également se trouver inversement en position 3' et 2', chaque nucléotide pouvant se trouver dans sa configuration D ou L et la base B pouvant se trouver en position α ou β.

2. Analogues d'oligonucléotides selon la revendication 1, **caractérisés en ce que** la base B se trouve en position β, les nucléotides se trouvent dans la configuration D, R² se trouve en position 2' et a représente oxy.

3. Analogues d'oligonucléotides selon la revendication 1 ou 2, **caractérisés en ce que**
R¹ signifie hydrogène, alkyle en C₁ à C₆, en particulier méthyle, ou un radical de formule II ;
R² signifie hydrogène ou hydroxy, en particulier hydrogène ;
d, e signifient un nombre entier de 5 à 15 ;
i signifie un nombre entier de 1 à 3 ;
m signifie un nombre entier de 1 à 6, en particulier 1 ;
U représente hydroxy, mercapto, alcoxy en C₁ à C₆, alkyle en C₁ à C₆, NR³R⁴ ou NHR³, en particulier hydroxy ou alkyle en C₁ à C₆,
où R³ représente alkyle en C₁ à C₈, de préférence alkyle en C₁ à C₄ ou méthoxyéthyle ;
et B, W, V, Y, Y', X et Z ont la signification susmentionnée.

4. Analogues d'oligonucléotides selon l'une ou plusieurs des revendications 1, 2 ou 3, **caractérisés en ce que** V, Y et Y' signifient oxy.

5. Analogues d'oligonucléotides selon l'une ou plusieurs des revendications 1 à 3, **caractérisés en ce que** W signifie oxo.

6. Analogues d'oligonucléotides selon l'une ou plusieurs des revendications 1 à 4 ou 5, **caractérisés en ce que** U signifie hydroxy.

7. Analogues d'oligonucléotides selon l'une ou plusieurs des revendications 1 à 4 ou 5, **caractérisés en ce que** R¹ représente hydrogène.

8. Procédé pour la préparation d'analogues d'oligonucléotides de formule IB selon la revendication 1, dans lequel
a) dans un premier cycle de réaction, on transforme une unité nucléotide comprenant un groupe de phosphore (V) en position terminale 3'(2') et un groupe 5'-hydroxy ou mercapto libre avec une autre unité nucléotide comprenant un groupe de phosphore (III) ou de phosphore (V) en position 3'(2') ou son dérivé activé ou avec un réactif qui permet l'introduction de groupements écarteurs, dans les autres cycles, on transforme une unité nucléotide avec un groupe de phosphore (III) ou de phosphore (V) en position terminale 3'(2') ou 5' ou son dérivé activé avec une autre unité nucléotide avec un groupe hydroxy ou mercapto libre en position terminale 5' ou 3'(2') ou
b) les analogues d'oligonucléotides sont construits par des fragments de la même manière,
on dissocie dans les oligonucléotides obtenus selon (a) ou (b) les groupes de protection introduits temporairement pour la protection d'autres fonctions et les analogues d'oligonucléotides ainsi obtenus de formule IB sont le cas échéant transformés en leurs sels physiologiquement acceptables.

9. Utilisation des analogues d'oligonucléotides selon l'une ou plusieurs des revendications 1 à 6 ou 7 pour la préparation d'un médicament destiné à l'inhibition de l'expression génique.

10. Utilisation des analogues d'oligonucléotides selon l'une ou plusieurs des revendications 1 à 6 ou 7 pour la préparation d'une sonde destinée à la détection d'acides nucléiques.

11. Préparation pharmaceutique contenant un ou plusieurs analogues d'oligonucléotides de formule IB selon l'une ou plusieurs des revendications 1-6 ou 7, le cas échéant avec des adjuvants et/ou des substances support physiologiquement acceptables.
